# EUROPEAN PATENT APPLICATION

(11) **EP 4 427 762 A1**
(43) Date of publication of application: **11.09.2024**
(21) Application number: 23161339.9
(22) Date of filing: 10.03.2023
(51) Int. Cl.: A61K 47/68, A61P 35/00

(54) **TRANS-CYCLOOCTENE WITH IMPROVED T-LINKER**

(71) Applicant: Tagworks Pharmaceuticals B.V., 6525 ED Nijmegen (NL)
(72) Inventor: ROSSIN, Raffaella, 6525 ED Nijmegen (NL); ROBILLARD, Marc Stefan, 6525 ED Nijmegen (NL); KLEIJN, Laurens Henri Johan, 6525 ED Nijmegen (NL)
(74) Representative: V.O.

(57) **Abstract**

Disclosed herein are *trans*-cyclooctenes (TCOs) that have an improved T-linker. In particular, the TCOs have improved *in vitro* and *in vivo* properties as compared to TCOs known from the prior art. The disclosure also pertains to *in vivo* and *in vitro* methods of using said *trans-*cyclooctenes, as well as medical uses thereof.

## Description

### Field of the invention

The invention disclosed herein relates to *trans*-cyclooctenes (TCOs) with improved properties. In particular, TCOs are provided that have a better clearance profile, a higher uptake at the target site, *e.g.* a tumor, a lower uptake off-target, and/or a cleaner metabolism profile as compared to known TCOs. Compositions and combinations comprising the TCOs of the invention, as well as methods for using same are provided as well.

### Background of the invention

In the field of bioorthogonal chemistry the ligation between TCOs and dienes, in particular tetrazines, is well-studied. While the ligation works well both *in vitro* as *in vivo,* it is still a challenge to find optimal compounds for clinical use. Regarding TCOs, a strong candidate for clinical use is considered to be compound **1**, disclosed in WO 2022/197182 as AVP0458-**22-PEG24**. Compound **1** is an AVP0458 diabody modified with four TCO-containing moieties, and has the following structure:

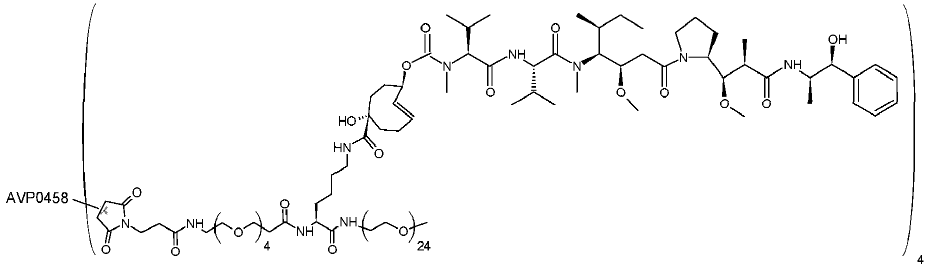

However, the inventors have identified, for the first time, several hitherto unknown disadvantages of compound **1**.

First, it was found that while compound **1** has a good clearance from blood, further improvements are desired. Compound **1** has a half-life in the blood of healthy mice of 4.22 hours, and 48 hours after injection in healthy mice 1.14% ID/g of compound **1** in the blood of said mice was observed. Based on this newly found disadvantage of compound **1**, it is desired that TCOs be provided that show faster clearance rates as compared to compound **1**.

Second, it was found that while compound **1** shows good tumor uptake of 18.42 %ID/g in LS174T xenograft bearing mice, further improvements are desired. It is therefore also desired that TCOs with a higher tumor uptake be provided.

Third, it was observed that compound **1** shows uptake at off-target sites, *e.g.* non-tumour sites such as the heart, lung, etc. It is also desired that TCOs be provided with a lower off-target uptake.

Fourth, it was observed that compound **1** shows a metabolism profile that can be improved. Thus, it is also desired that TCOs be provided showing a better metabolism profile.

Finally, it is desired that TCOs be provided with overall good *in vitro* and *in vivo* properties, *i.e.* one or more of: good stability, good reactivity with tetrazines, and/or high payload release (especially *in vivo*)*.*

There is thus a need for TCOs that address one or more of the abovementioned problems and/or desires.

### Summary of the invention

The invention relates to at least the following embodiments:
1. A compound or a salt, hydrate, or solvate thereof; wherein said compound has a structure according to Formula (1): 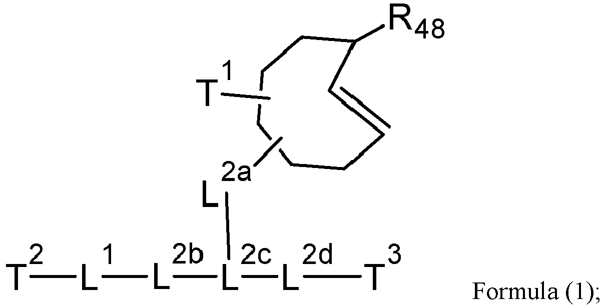 wherein
   **L¹** is selected from the group consisting of linear or branched C₄-C₁₂ alkylene, C₃-C₈ (hetero)cycloalkylene, C₆-C₁₂ arylene, and C₄-C₁₁ heteroarylene;
   **L^{2a}**, **L^{2b}**, and **L^{2d}** are each independently a linker;
   **L^{2c}** is selected from the group consisting of C₁-C₈ (hetero)alkanetriyl, C₅-C₆ (hetero)arenetriyl. C₃-C₇ cycloalkanetriyl, and C₂-C₇ heterocycloalkanetriyl;
   **T¹** is selected from the group consisting of -OT^{1A}, hydrogen, C₂-C₆ alkyl, C₆ aryl, C₄-C₅ heteroaryl, C₃-C₆ cycloalkyl, C₅-C₁₂ alkyl(hetero)aryl, C₅-C₁₂ (hetero)arylalkyl, C₄-C₁₂ alkylcycloalkyl, -N(T^{1A})₂, -ST^{1A}, -SO₃H, -C(O)T^{1A}, -C(O)OT^{1A}, -O-C(O)T^{1A} -C(O)N(T^{1A})₂, -N(T^{1A})₂-CO-T^{1A}, and -Si(T^{1A})₃;
      each **T^{1A}** is independently selected from the group consisting of hydrogen, (hetero)alkyl, (hetero)alkenyl, (hetero)alkynyl, (hetero)aryl, and an amino acid residue;
   **T²** is a bioconjugation moiety or a group -L³-C^{B}; wherein
      **L³** is a residue of a bioconjugation moiety, and
      **C^{B}** is selected from the group consisting of proteins, nucleic acids, peptides, carbohydrates, aptamers, lipids, small organic molecules, polymers, LNA, PNA, amino acids, peptoids, chelating moieties, fluorescent dyes, phosphorescent dyes, organic particles, gels, cells, and combinations thereof;
   **T³** is a polymer; and
   **R₄₈** is selected from the group consisting of -OH, -O-acetyl, -O-C₁₋₄ alkyl, halogen, active carbonate, and a releasable group; and
      preferably **L¹** is linear or branched C₄-C₁₂ alkylene, more preferably **L¹** is linear or branched C₄-C₁₀ alkylene, and most preferably L¹ is linear C₅-C₆ alkylene;
      preferably L^{2a}, L^{2b}, and L^{2d} are each independently a linker containing at most twenty atoms; more preferably L^{2a}, L^{2b}, and L^{2d} are each independently selected from the group consisting of -C(O)NL^{2T}-, -NL^{2T}C(O)-, -O-, -S-, -NL^{2T}-, -N=N-, and -C(O)-; wherein L^{2T} is hydrogen or methyl, preferably L^{2T} is hydrogen;
      preferably L^{2c} is C₁-C₈ (hetero)alkanetriyl, more preferably L^{2c} is C₁-C₈ alkanetriyl, and most preferably L^{2c} is C₄-C₆ alkanetriyl;
      preferably T¹ is -OT^{1A}; and most preferably T¹ is -OH;
      preferably T^{1A} is hydrogen or methyl, more preferably T^{1A} is hydrogen;
      preferably T² is maleimidyl, N-hydroxysuccinimidyl, or -L³-C^{B};
      preferably L³ is a residue of a maleimidyl moiety or a residue of an N-hydroxysuccinimidyl moiety;
      preferably C^{B} is a protein, more preferably C^{B} is an antibody or a diabody, even more preferably C^{B} is a diabody, and most preferably C^{B} is AVP0458 according to SEQ ID NO: 1;
      preferably T³ is a polymer comprising a polyethylene glycol moiety; and
      preferably R₄₈ is a releasable group.
2. The compound according to Embodiment 1, or a salt, hydrate, or solvate thereof; wherein said compound is according to Formula (2): 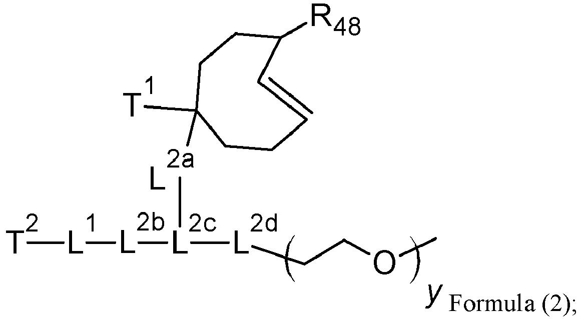 wherein y is an integer in a range of from 1 to 50;
   preferably y is an integer in a range of from 10 to 40, more preferably in a range of from 12 to 37, even more preferably in a range of from 15 to
   35, more preferably still in a range of from 20 to 30, and most preferably in a range of from 23 to 25.
3. The compound according to any one of the preceding Embodiments, or a salt, hydrate, or solvate thereof; wherein said compound is according to Formula (3): 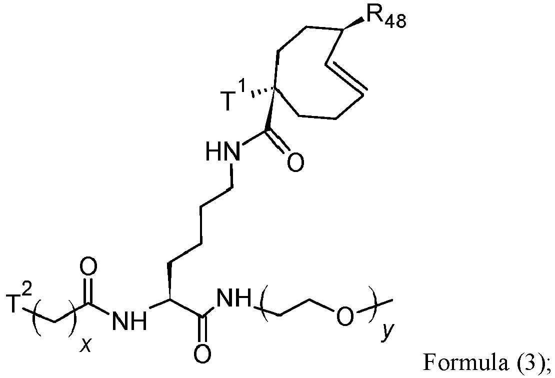 wherein
   y is as defined in Embodiment 2;
   x is an integer in a range of from 4 to 12;
      preferably x is an integer in a range of from 4 to 8, more preferably x is an integer in a range of from 4 to 6.
4. The compound according to any one of the preceding Embodiments, or a salt, hydrate, or solvate thereof; wherein R₄₈ is a releasable group, and said releasable group is -O-CO-C^{A}; wherein C^{A} is a drug;
   preferably the drug is linked to the moiety -O-CO- via a secondary or tertiary nitrogen atom that is part of the drug, forming a carbamate;
   preferably the drug is monomethyl auristatin E (MMAE).
5. The compound according to any one of the preceding Embodiments, or a salt, hydrate, or solvate thereof; wherein
   T² is 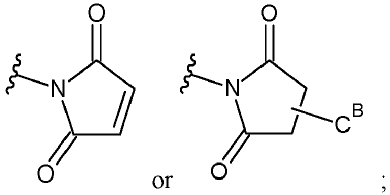 and
   C^{B} is a protein;
      preferably C^{B} is an antibody or a diabody, more preferably a diabody, and most
      preferably AVP0458 according to SEQ ID NO: 1.
6. The compound according to any one of the preceding Embodiments, or a salt, hydrate, or solvate thereof; wherein said compound is: 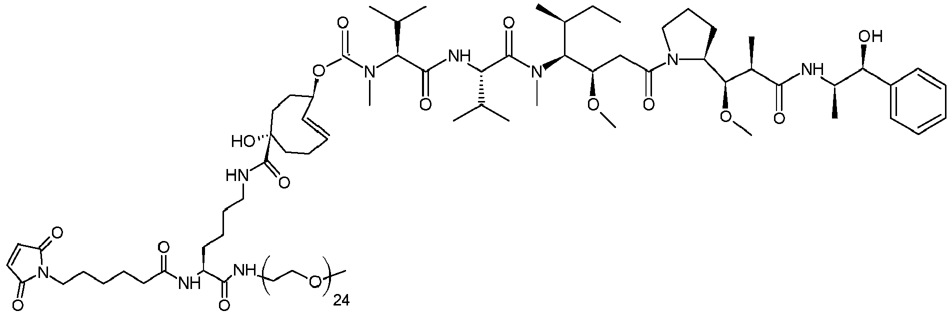
7. The compound according to any one of the preceding Embodiments, or a salt, hydrate, or solvate thereof; wherein said compound is 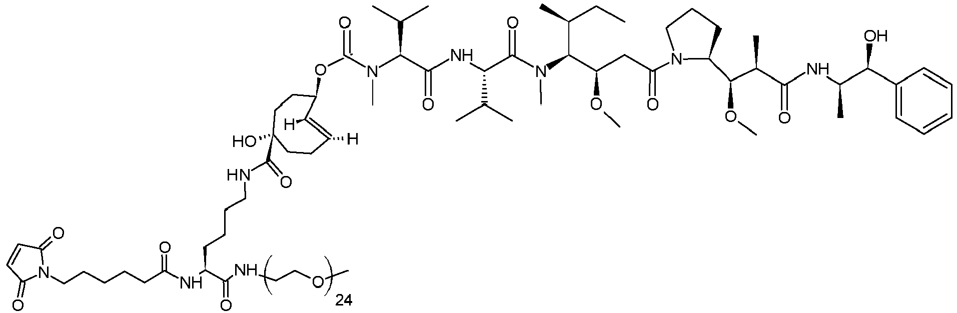 or 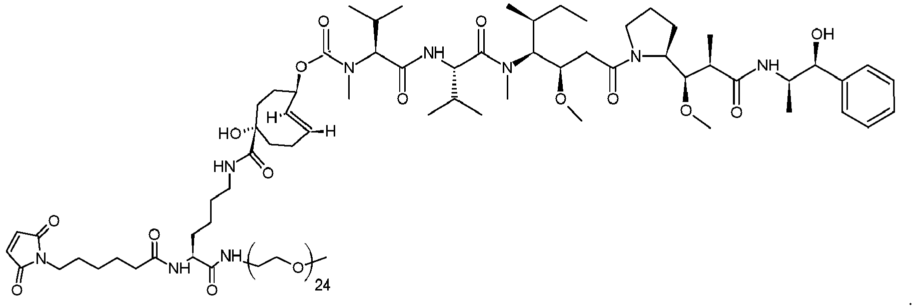
8. The compound according to any one of Embodiments 1 to 5, or a salt, hydrate, or solvate thereof; wherein said compound is: 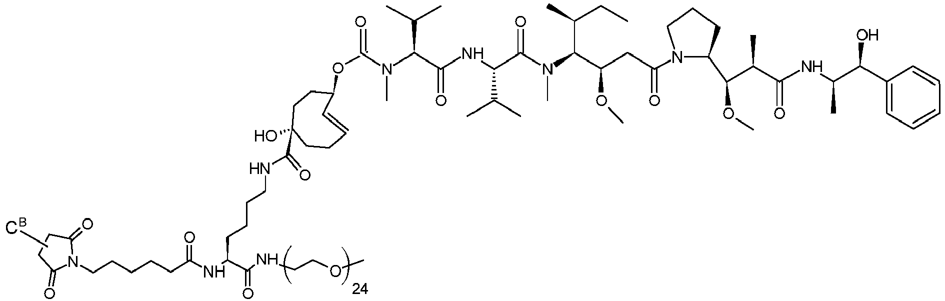 wherein C^{B} is AVP0458 according to SEQ ID NO: 1;
   preferably C^{B} is linked to the maleimidyl group via a sulfur atom, preferably the sulfur atom is part of a cysteine.
9. The compound according to Embodiment 8, or a salt, hydrate, or solvate thereof; wherein said compound is: 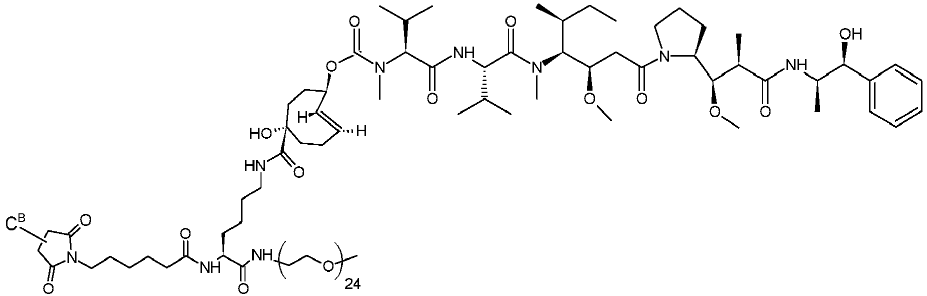 or 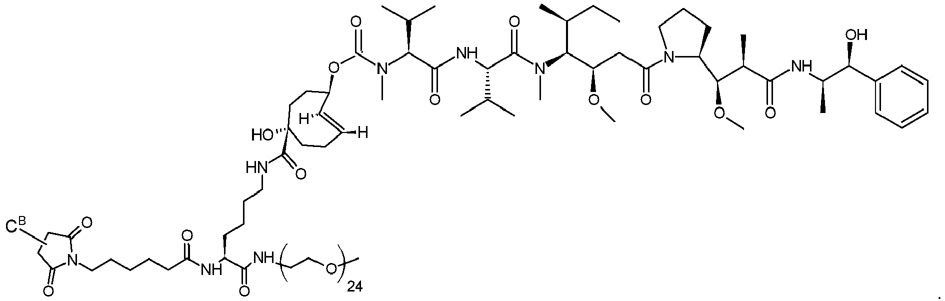
10. A compound or a salt, hydrate, or solvate thereof; wherein said compound comprises an eight-membered non-aromatic cyclic mono-alkenylene moiety, wherein said moiety comprises a non-vinylic carbon atom, wherein said non-vinylic carbon atom is substituted with at least one structure according to Formula (A): 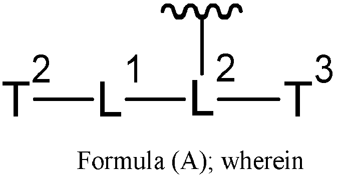 wherein
   L¹ and L² are each independently a linker; and
   T² and T³ are organic moieties.
11. A conjugate, or a salt, hydrate, or solvate thereof, wherein the conjugate comprises a protein conjugated to at least one compound according to Formula (1) as defined in any one of Embodiments 1 to 9, wherein L¹, L^{2a}, L^{2b}, L^{2c}, L^{2d}, T¹, T³, and R⁴⁸ are as defined in any one of Embodiments 1 to 9, and wherein T² is a residue of a bioconjugation moiety, and said protein and said compound are conjugated via T²;
   preferably the protein is a diabody or an antibody; more preferably the protein is a diabody; and most preferably the protein is AVP0458 according to SEQ ID NO: 1;
   preferably the protein is conjugated to at most 12 of said compounds; more
   preferably the protein is conjugated to at most 8 of said compounds, most
   preferably the protein is conjugated to at most 4 of said compounds;
   preferably said protein and said compound are conjugated via T² and a residue of a sulfhydryl of said protein, a residue of a hydroxyl of said protein, or a residue of an amine of said protein; more preferably said protein and said compound are conjugated via T² and a residue of a sulfhydryl of said protein;
   preferably T² is a residue of a maleimidyl moiety or a residue of an N-hydroxysuccinimidyl moiety; more preferably T² is a residue of a maleimidyl moiety.
12. The conjugate according to Embodiment 11, or a salt, hydrate, or solvate thereof, wherein the conjugate is 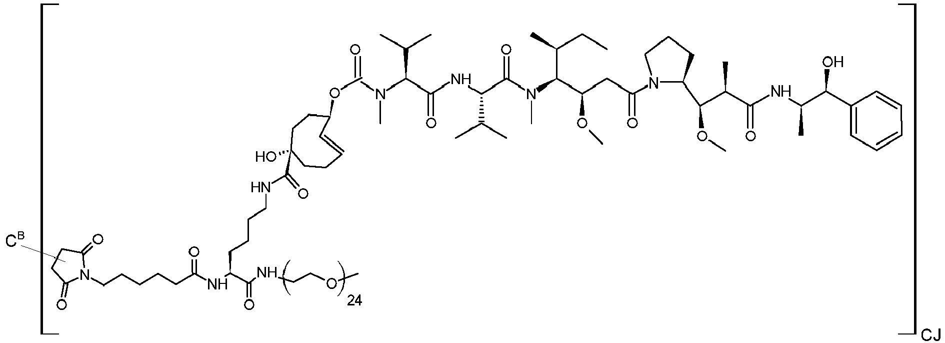
   wherein CJ is in a range of from 1 to 12;
   wherein C^{B} is AVP0458 according to SEQ ID NO: 1;
      preferably CJ is of from 2 to 10, more preferably of from 2.5 to 8, even more
      preferably of from 3 to 6, even more preferably still of from 3.5 to 4, and most
      preferably about 4;
      preferably C^{B} is linked to each maleimidyl group via a sulfur atom, preferably the sulfur atom is part of a cysteine.
13. The conjugate according to Embodiment 12, or a salt, hydrate, or solvate thereof, wherein the conjugate is 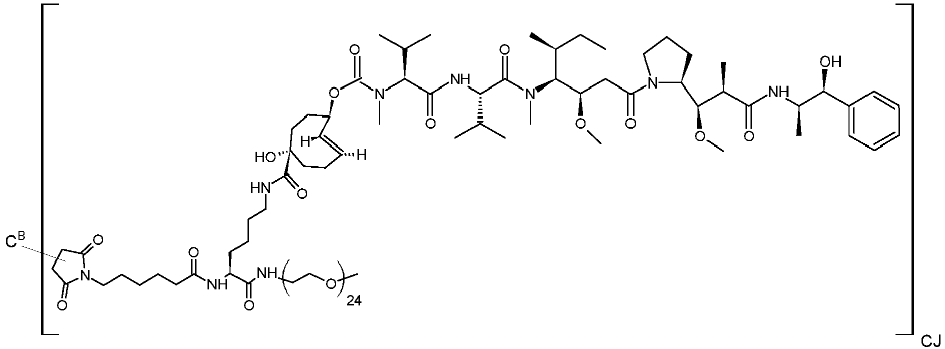 or 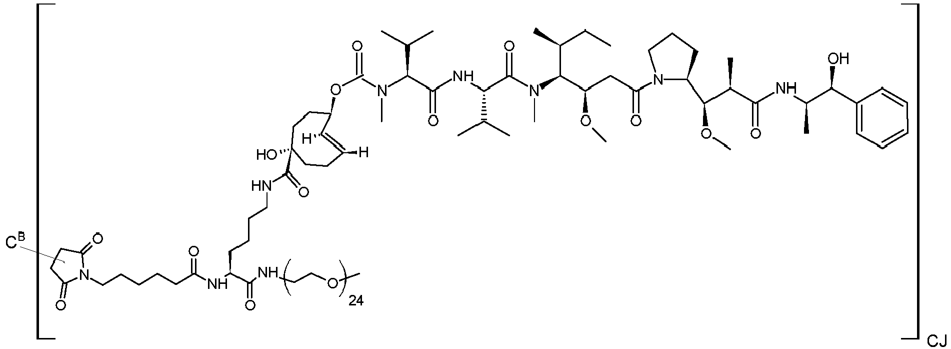
14. A composition comprising:
   (a) a compound according to any one of Embodiments 1 to 10, or the salt, hydrate, or solvate thereof; and/or
   (b) the conjugate according to any one of Embodiments 11 to 13, or the salt, hydrate, or solvate thereof;
      preferably the composition is a pharmaceutical composition.
15. A composition according to Embodiment 14, wherein said composition comprises:
   (a) a compound according to any one of Embodiments 1 to 10, or the salt, hydrate, or solvate thereof; and
   (b) the enantiomer of said compound, or the salt, hydrate, or solvate thereof;
      preferably said composition is a racemic mixture of (a) and (b).
16. A combination of
   (A1) a compound according to any one of Embodiments 1 to 10, or the salt, hydrate, or solvate thereof;
   (A2) a conjugate according to any one of Embodiments 11 to 13, or the salt, hydrate, or solvate thereof; and/or
   (A3) a composition according to Embodiment 14 or 15: with
   (B) a diene or a salt, solvate, or hydrate thereof;
      preferably the diene is a tetrazine.
17. The combination according to Embodiment 16, wherein the diene is 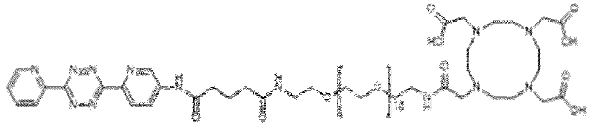 ; or a salt, hydrate, and/or solvate thereof.
18. The compound according to any one of Embodiments 1 to 10, or the salt, hydrate, or solvate thereof; the conjugate according to any one of Embodiments 11 to 13, or the salt, hydrate, or solvate thereof; the composition according to any one of Embodiments 14 to 15; or the combination according to any one of Embodiments 16 to 17; for use as a medicament.
19. The compound according to any one of Embodiments 1 to 10, or the salt, hydrate, or solvate thereof; the conjugate according to any one of Embodiments 11 to 13, or the salt, hydrate, or solvate thereof; the composition according to any one of Embodiments 14 to 15; or the combination according to any one of Embodiments 16 to 17; for use in the treatment of a disease in a subject,
   preferably the subject is a human;
   preferably the disease is cancer.
20. A method of treating a disease in a subject, wherein said method comprises the step of administering to said subject:
   (a) the compound according to any one of Embodiments 1 to 10, or the salt, hydrate, or solvate thereof;
   (b) the conjugate according to any one of Embodiments 11 to 13, or the salt, hydrate, or solvate thereof;
   (c) the composition according to any one of Embodiments 14 to 15; and/or
   (d) the combination according to any one of Embodiments 16 to 17;
      preferably the subject is a human;
      preferably the disease is cancer.
21. A non-therapeutic method for reacting:
   (ia) the compound according to any one of Embodiments 1 to 10, or the salt, hydrate, or solvate thereof;
   (iia) the conjugate according to any one of Embodiments 11 to 13, or the salt, hydrate, or solvate thereof; and/or
   (iiia) the composition according to any one of Embodiments 14 to 15;
   with a diene or a salt, solvate, or hydrate thereof,
   wherein said method comprises the step of contacting (ia), (iia), or (iiia) with said diene or salt, solvate, or hydrate thereof,
      preferably said non-therapeutic method is an *in vitro* method; and
      preferably said diene is a tetrazine.
22. A non-therapeutic use of:
   (a) the compound according to any one of Embodiments 1 to 10, or the salt, hydrate, or solvate thereof;
   (b) the conjugate according to any one of Embodiments 11 to 13, or the salt, hydrate, or solvate thereof;
   (c) the composition according to any one of Embodiments 14 to 15; and/or
   (d) the combination according to any one of Embodiments 16 to 17;
   in a click reaction.

### Detailed Description of the Invention

The invention, in a broad sense, is based on the judicious insight that TCOs of the invention meet one or more of the aforementioned desires. In particular, it was surprisingly found that replacing the PEG4 moiety of compound **1** resulted in a higher clearance rate, higher tumor uptake, lower off-target uptake, a better metabolism profile, and/or further improved *in vitro* and *in vivo* properties.

Especially the combination of a faster clearance rate and a higher tumor uptake is surprising, since this means that the faster clearance from blood is not due to *e.g.* excretion from the body. Instead, the compounds of the invention are quickly taken up in the tumour. Even more advantageously the uptake of compounds of the invention in off-target tissues is much lower as compared to the uptake in the tumour. This means that a higher percentage of payload can be released at the desired target site, and that the trigger to activate this payload release (typically a diene, *e.g.* a tetrazine) can be administered at an earlier point in time, shortening the entire procedure. Thus, the compounds of the invention also result in a higher convenience for patients, as fewer and/or less severe side-effects are expected as well as a shorter treatment time.

Preferred embodiments of the invention are further described below. All of these embodiments can be combined as long as they are not mutually exclusive.

### Compounds of the invention

The compounds of the invention are according to Formula (1) as defined above. For ease of reference, compounds of Formula (1) are below described as *e.g*. "compounds of the invention". It will be understood that also the salt, hydrate, or solvate of said compounds are included by such a statement.

### AVP0458

As used herein, AVP0458 refers to a TAG72-binding diabody derived from the CC49 antibody. AVP0458 is a diabody having an amino acid sequence according to SEQ ID NO:1:

### SEQ ID NO:1 (amino acid sequence of AVP0458 diabody):

SVQLQQSDAELVKPGASVKISCKASGYTFTDHAIHWVKQNPEQGLEWIGYFSPGNDD
FKYNERFKGKATLTADKSSSTAYLQLNSLTSEDSAVYFCTRSLNMAYWGQGTSVTV
SSGGGGSDIVMTQSCSSCPVSVGEKVTLSCKSSQSLLYSGNQKNYLAWYQQKPGQSP
KLLIYWASTRESGVPDRFTGSGSGTDFTLSISSVETEDLAVYYCQQYYSYPLTFGAGT
KLVLKR

### Bioconjugation moieties

In Formula (1), T² can be a bioconjugation moiety. Preferably, the bioconjugation moiety is selected from the group consisting of N-maleimidyl, halogenated N-alkylamido, sulfonyloxy N-alkylamido, vinyl sulfone, (activated) carboxylic acids, benzenesulfonyl halides, ester, carbonate, sulfonyl halide, thiol or derivatives thereof, C₂₋₆ alkenyl, C₂₋₆ alkynyl, C₇₋₁₈ cycloalkynyl, C₅₋₁₈ heterocycloalkynyl, bicyclo[6.1.0]non-4-yn-9-yl], C₃₋₁₂ cycloalkenyl, azido, phosphine, nitrile oxide, nitrone, nitrile imine, isonitrile, diazo, ketone, (O-alkyl)hydroxylamino, hydrazine, halogenated N-maleimidyl, aryloxymaleimides, dithiophenolmaleimides, bromo- and dibromopyridazinediones, 2,5-dibromohexanediamide, alkynone, 3-arylpropiolonitrile, 1,1-bis(sulfonylmethyl)-methylcarbonyl or elimination derivatives thereof, carbonyl halide, allenamide, 1,2-quinone, isothiocyanate, isocyanate, aldehyde, triazine, squaric acids, 2-imino-2-methoxyethyl, (oxa)norbornene, (imino)sydnones, methylsulfonyl phenyloxadiazole, aminooxy, 2-amino benzamidoxime, ethynylphosphonamidates, reactive in the Pictet-Spengler ligation and hydrazine-Pictet-Spengler (HIPS) ligation, DNA intercalators, tetrazine, *trans*-cyclooctene, and photocrosslinkers. More preferably, the bioconjugation moiety is N-maleimidyl. Residues of these bioconjugation moieties are known in the art.

### Releasable groups

In Formula (1), R⁴⁸ is preferably a releasable group. Such releasable groups are well-known and have a clear meaning in the art.

In preferred embodiments, the releasable group is -(Y¹-C(=Y²))ᵢ-(S^{p})ⱼ-C^{A}. Therein, each of Y¹ and Y² are independently selected from O, and S; preferably Y¹ and Y² are O. C^{A} is Construct A, which is the payload. Preferably, C^{A} is an organic molecule or an inorganic molecule. Further preferred embodiments of C^{A} are defined below. For the releasable group, j is 0 or 1; preferably j is 0; and i is 0 or 1; preferably i is 1. If i is 0, -(S^{P})ⱼ-C^{A} is connected to the remainder of the compound via O or S, that is part of -(S^{P})ⱼ-C^{A}. On the other hand, if i is 1, -(S^{P})ⱼ-C^{A} is connected to -C(=Y²)- via O, S, a secondary N, or a tertiary N, that is part of -(S^{P})ⱼ-C^{A}. Preferably, if i is 1, -(S^{P})ⱼ-C^{A} is connected to -C(=Y²)- via a secondary N, or a tertiary N, that is part of -(S^{P})ⱼ-C^{A}.

S^{P} is a spacer, of which preferred embodiments are defined below. Preferably, when S^{P} is part of a releasable group, S^{P} is a self-immolative linker, which is herein also referred to as L^{C}. Such self-immolative linkers are well-known in the art, and preferred embodiments of self-immolative linkers are defined below. If the spacer in the releasable group is a self-immolative linker, upon reaction of a compound of the invention with a diene, initially a construct -L^{C}-C^{A} is released. Thereafter, the self-immolative linker self-immolates and releases the payload C^{A}.

### Spacers S^{P}

All linkers as used herein may each independently be a spacer S^{P}.

As the skilled person is aware, the specific structure of a spacer used in either a dienophile or diene as described herein does not typically influence whether the payload is released. However, in some cases specific spacers are preferred. For example, if a payload is to be released, the spacer between *e.g*. the allylic carbon of the (*E*)-bicyclo[6.1.0]non-3-ene moiety and the payload is preferably a self-immolative linker. Such a linker, which is typically referred to as L^{C} herein, ensures that upon release of the end of the linker connected to said allylic carbon, a further rearrangement or reaction takes place, after which the payload is decoupled from the linker L^{C}. Below, first spacers in general are discussed, and thereafter the more specific self-immolative linkers.

In general, a spacer S^{P} as used herein is a moiety according to RG2, more preferably any one of the preferred and/or specific embodiments thereof.

Preferably, a spacer S^{P} consists of one or multiple Spacer Units S^{U} arranged linearly and/or branched and may be connected to one or more C^{B} moieties and/or one or more L^{C} or T^{R} moieties. The Spacer may be used to connect C^{B} to one T^{R} (Example A below; with reference to Formula 5a and 5b: f, e, a = 1) or more T^{R} (Example B and C below; with reference to Formula 5a and 5b: f, e = 1, a ≥ 1), but it can also be used to modulate the properties, e.g. pharmacokinetic properties, of the C^{B}-T^{R}-C^{A} conjugate (Example D below; with reference to Formula 5a and 5b: one or more of c,e,g,h ≥ 1). Thus a Spacer unit does not necessarily connect two entities together, it may also be bound to only one component, e.g. the T^{R} or L^{C}. Alternatively, the Spacer may comprise a Spacer Unit linking C^{B} to T^{R} and in addition may comprise another Spacer Unit that is only bound to the Spacer and serves to modulate the properties of the conjugate (Example F below; with reference to Formula 5a and 5b: e ≥ 1). The Spacer may also consist of two different types of S^{U} constructs, e.g. a PEG linked to a peptide, or a PEG linked to an alkylene moiety (Example E below; with reference to Formula 5a and 5b: e ≥ 1). For the sake of clarity, Example B depicts a S^{U} that is branched by using a multivalent branched S^{U}. Example C depicts a S^{U} that is branched by using a linear S^{U} polymer, such as a peptide, whose side chain residues serve as conjugation groups. 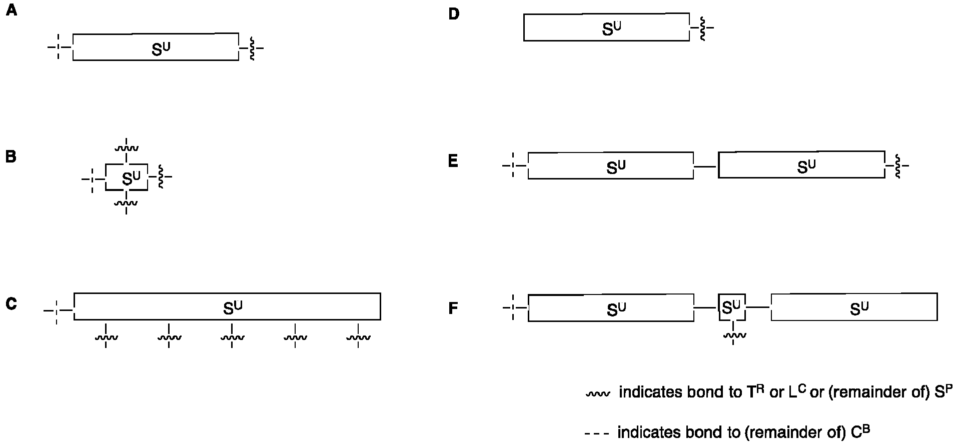
A
S^U

B
S^U

C
S^U

D
S^U

E
S^U
S^U

F
S^U
S^U
S^U

indicates bond to T^R or L^C or (remainder of) S^P

- - - indicates bond to (remainder of) C^B
 The Spacer may be bound to the Activator in similar designs such as depicted in above examples A- F.

Each individual spacer unit S^{U} may be independently selected from the group of radicals according to RG2. The Spacer Units include but are not limited to amino acids, nucleosides, nucleotides, and biopolymer fragments, such as oligo- or polypeptides, oligo- or polypeptoids, or oligo- or polylactides, or oligo- or poly-carbohydrates, varying from 2 to 200, particularly 2 to 113, preferably 2 to 50, more preferably 2 to 24 and more preferably 2 to 12 repeating units. Preferred biopolymer S^{U} are peptides. Preferably each S^{U} comprises at most 50 carbon atoms, more preferably at most 25 carbon atoms, more preferably at most 10 carbon atoms. In some embodiments the S^{U} is independently selected from the group consisting of (CH₂)ᵣ, (C₃-C₈ carbocyclo), O-(CH₂)ᵣ, arylene, (CH₂))-arylene, arylene-(CH₂)ᵣ, (CH₂)ᵣ-(C₃-C₈ carbocyclo), (C₃-C₈ carbocyclo)-(CH₂)ᵣ, (C₃-C₈ heterocyclo), (CH₂)ᵣ-(C₃-C₈ heterocyclo), (C₃-C₈ heterocyclo)-(CH₂)ᵣ, -(CH₂)ᵣC(O)NR'(CH₂)ᵣ, (CH₂CH₂O)ᵣ, (CH₂CH₂O)ᵣCH₂,(CH₂)ᵣC(O)NR'(CH₂ CH₂O)ᵣ, (CH₂)ᵣC(O)NR'(CH₂CH₂O)ᵣCH₂, (CH₂CH₂O)ᵣ C(O)NR'(CH₂CH₂O)ᵣ, (CH₂CH₂O)ᵣC(O)NR'(CH₂CH₂O)ᵣCH₂, (CH₂CH₂O)ᵣC(O)NR'CH₂; wherein r is independently an integer from 1 -10. As used herein, each R'is independently selected from the group consisting of radicals according to RG1. Preferably, R'is hydrogen. Other examples of Spacer Units S^{U} are linear or branched polyalkylene glycols such as polyethylene glycol (PEG) or polypropylene glycol (PPG) chains varying from 2 to 200, particularly 2 to 113, preferably 2 to 50, more preferably 2 to 24 and more preferably 2 to 12 repeating units. It is preferred that when polyalkylene glycols such as PEG and PPG polymers are only bound via one end of the polymer chain, that the other end is terminated with -OCH₃, -OCH₂CH₃, OCH₂CH₂CO₂H. Other polymeric Spacer Units are polymers and copolymers such as poly-(2-oxazoline), poly(*N*-(2-hydroxypropyl)methacrylamide) (HPMA), polylactic acid (PLA), polylactic-glycolic acid (PLGA), polyglutamic acid (PG), dextran, polyvinylpyrrolidone (PVP), poly(1-hydroxymethylethylene hydroxymethyl-formal (PHF). Other exemplary polymers are polysaccharides, glycopolysaccharides, glycolipids, polyglycoside, polyacetals, polyketals, polyamides, polyethers, polyesters. Examples of naturally occurring polysaccharides that can be used as S^{U} are cellulose, amylose, dextran, dextrin, levan, fucoidan, carraginan, inulin, pectin, amylopectin, glycogen, lixenan, agarose, hyaluronan, chondroitinsulfate, dermatansulfate, keratansulfate, alginic acid and heparin. In yet other exemplary embodiments, the polymeric S^{U} comprises a copolymer of a polyacetal/polyketal and a hydrophilic polymer selected from the group consisting of polyacrylates, polyvinyl polymers, polyesters, polyorthoesters, polyamides, oligopeptides, polypeptides and derivatives thereof. Preferred polymeric S^{U} are PEG, HPMA, PLA, PLGA, PVP, PHF, dextran, oligopeptides, and polypeptides. In some embodiments, polymers used in a S^{U} have a molecular weight ranging from 2 to 200 kDa, from 2 to 100 kDa, from 2 to 80 kDa, from 2 to 60 kDa, from 2 to 40 kDa, from 2 to 20 kDa, from 3 to 15 kDa, from 5 to 10 kDa, from 500 dalton to 5 kDa. Other exemplary S^{U} are dendrimers, such as polypropylene imine) (PPI) dendrimers, PAMAM dendrimers, and glycol-based dendrimers. The S^{U} of the invention expressly include but are not limited to conjugates prepared with commercially available cross-linker reagents such as BMPEO, BMPS, EMCS, GMBS, HBVS, LC-SMCC, MBS, MPBH, SBAP, SIA, SIAB, SMCC, SMPB, SMPH, sulfo-EMCS, sulfo-GMBS, sulfo-KMUS, sulfo-MBS, sulfo-SIAB, sulfo-SMCC, sulfo-SMPB, and SVSB, DTME, BMB, BMDB, BMH, BMOE, BM(PEO)₃ and BM(PEO)₄. To construct a branching Spacer one may use a S^{U} based on one or several natural or non-natural amino acids, amino alcohol, aminoaldehyde, or polyamine residues or combinations thereof that collectively provide the required functionality for branching. For example serine has three functional groups, i.e. acid, amino and hydroxyl groups and may be viewed as a combined amino acid an aminoalcohol residue for purpose of acting as a branching S^{U}. Other exemplary amino acids are lysine and tyrosine. In some embodiments, the Spacer consists of one Spacer Unit, therefore in those cases S^{P} equals S^{U}. Preferably the Spacer consists of two, three or four Spacer Units. In some embodiments, S^{P} has a molecular weight ranging from 2 to 200 kDa, from 2 to 100 kDa, from 2 to 80 kDa, from 2 to 60 kDa, from 2 to 40 kDa, from 2 to 20 kDa, from 3 to 15 kDa, from 5 to 10 kDa, or from 500 dalton to 5 kDa. In some embodiments, the S^{P} has a mass of no more than 5000 daltons, no more than 4000 daltons, no more than 3000 daltons, no more than 2000 daltons, no more than 1000 daltons, no more than 800 daltons, no more than 500 daltons, no more than 300 daltons, no more than 200 daltons. In some aspects the S^{P} has a mass from 100 daltons, from 200 daltons, from 300 daltons to 5000 daltons. In some aspects of the S^{P} has a mass from 30, 50, or 100 daltons to 1000 daltons, from about 30, 50, or 100 daltons to 500 daltons.

Preferably, S^{P} comprises a moiety RG2a, RG2b, RG2c, or a residue of RG1f, as described herein. Preferably, said RG2a, RG2b, RG2c, or a residue of RG1f connects the S^{P} to C^{B}, L^{C}, or T^{R}.

### Self-immolative linkers L^{C}

L^{C} is an optional self-immolative linker, which may consist of multiple units arranged linearly and/or branched. The possible L^{C} structures, their use, position and ways of attachment of linkers L^{C}, C^{A} and the T^{R} are known to the skilled person, see for example [Papot et al., Anticancer Agents Med. Chem., 2008, 8, 618-637]. Nevertheless, preferred but non-limiting examples of self-immolative linkers L^{C} are benzyl-derivatives, such as those drawn below. There are two main self-immolation mechanisms: electron cascade elimination and cyclization-mediated elimination. The preferred example below on the left functions by means of the cascade mechanism, wherein the bond between the allylic carbon of the Trigger and the -O- or -S- attached to said carbon is cleaved, and an electron pair of Y^{C1}, for example an electron pair of NR⁶, shifts into the benzyl moiety resulting in an electron cascade and the formation of 4-hydroxybenzyl alcohol, CO₂ and the liberated payload. The preferred example in the middle functions by means of the cyclization mechanism, wherein cleavage of the bond to the NR⁶ on the side of the Trigger leads to nucleophilic attack of the amine on the carbonyl, forming a 5-ring 1,3-dimethylimidazolidin-2-one and liberating the payload. The preferred example on the right combines both mechanisms. This linker will degrade not only into CO₂ and one unit of 4-hydroxybenzyl alcohol (when Y^{C1} is O), but also into one 1,3-dimethylimidazolidin-2-one unit. 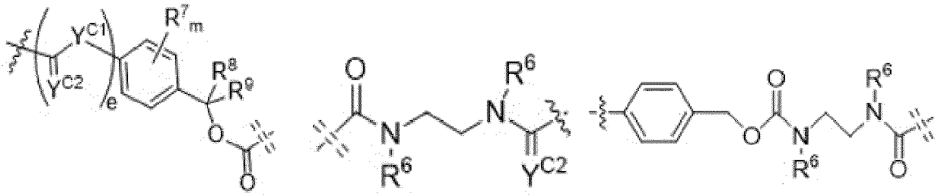 wherein the wiggly line indicates a bond to -O- or -S- on the allylic position of the trans-cyclooctene, and the double dashed line indicates a bond to C^{A}.

By substituting the benzyl groups of aforementioned self-immolative linkers L^{C}, it is possible to tune the rate of release of the payload, caused by either steric and/or electronic effects on the cyclization and/or cascade release. Synthetic procedures to prepare such substituted benzyl-derivatives are known to the skilled person (see for example [Greenwald et al, J. Med. Chem., 1999, 42, 3657-3667] and [Thornthwaite et al, Polym. Chem., 2011, 2, 773-790]. Some preferred substituted benzyl-derivatives with different release rates are drawn below.

Self-immolative linkers that undergo cyclization include but are not limited to substituted and unsubstituted aminobutyric acid amide, appropriately substituted bicyclo[2.2.1] and bicyclo[2.2.2] ring system, 2-aminophenylpropionic acid amides, and trimethyl lock-based linkers, see e.g. [Chem. Biol. 1995, 2, 223], [J. Am. Chem. Soc. 1972, 94, 5815], [J. Org. Chem. 1990, 55, 5867], the contents of which are hereby incorporated by reference. Further preferred examples of L^{C} can be found in WO2009017394(A1), US7375078, WO2015038426A1, WO2004043493, Angew. Chem. Int. Ed. 2015, 54, 7492 - 7509, the contents of which are hereby incorporated by reference.

Preferably the L^{C} has a mass of no more than 1000 daltons, no more than 500 daltons, no more than 400 daltons, no more than 300 daltons, or from 10, 50 or 100 to 1000 daltons, from 10, 50, 100 to 400 daltons, from 10, 50, 100 to 300 daltons, from 10, 50, 100 to 200 daltons, e.g., 10-1000 daltons, such as 50-500 daltons, such as 100 to 400 daltons.

A person skilled in the art will know that one L^{C} may be connected to another L^{C} that is bound to C^{A}, wherein upon reaction of the Activator with the Trigger T^{R}, L^{C}-L^{C}-C^{A} is released from the T^{R}, leading to self-immolative release of both L^{C} moietes and the payload. With respect to the L^{C} formulas disclosed herein, the L^{C} linking the T^{R} to the other L^{C} then does not release the payload but an L^{C} that is bound via Y^{C1} and further links to C^{A}. The skilled person will acknowledge that this principle also holds for further linkers L^{C} linked to L^{C}, *e.g.* L^{C}-L^{C}-L^{C}-L^{C}-C^{A}.

Preferably, if the releasable group contains a self-immolative linker, the releasable group is according to any one of Group I, Group II, Group III, and Group IV as shown below. In the structures depicted for said Groups, only bonds to Construct A and an atom (typically oxygen) on the allylic position of the trans-cyclooctene ring (part of the (*E*)-bicyclo[6.1.0]non-3-ene moiety) are shown for reasons of clarity, but said Construct A and said atom are part of the releasable group.

Releasable groups according to Group I are

| | |
|---|---|
| 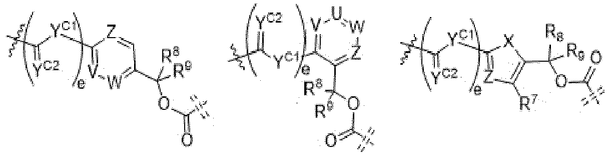 | 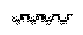 Indicates bond to -O- on the allylic position of the trans-cyclooctene ring |
| | 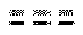 indicates bond to C^{A} |

, wherein the wiggly line may also indicate a bond to -S- on the allylic position of the trans-cyclooctene, wherein U, V, W, Z are each independently selected from the group consisting of -CR⁷-, and -N-, wherein e is 0 or 1, wherein X is selected from the group consisting of -O-, -S- and -NR⁶-, wherein preferably each R⁸ and R⁹ are independently selected from the group consisting of hydrogen, C₁-C₄ (hetero)alkyl, C₂-C₄ (hetero)alkenyl, and C₄₋₆ (hetero)aryl; wherein for R⁸ and R⁹ the (hetero)alkyl, (hetero)alkenyl, and (hetero)aryl are optionally substituted with a moiety selected from the group consisting of -Cl, -F, -Br, -I, -OH, -NH₂, =O, -SH, -SO₃H, -PO₃H -PO₄H₂ and -NO₂ and preferably contain at most two heteroatoms selected from the group consisting of -O-, -S-, -NH-, -P-, and -Si-, wherein the N, S, and P atoms are optionally oxidized. Preferably, for releasable groups of Group I both R⁸ and R⁹ are hydrogen.

The releasable group according to Group II is

| | |
|---|---|
| 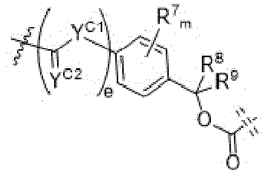 | 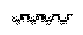 indicates bond to -O- on the allylic position of the trans-cyclooctene ring |
| | 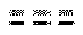 indicates bond to C^{A} |

, wherein the wiggly line may also indicate a bond to -S- on the allylic position of the trans-cyclooctene, wherein m is an integer between 0 and 2, preferably m is 0, wherein e is 0 or 1. Preferably, for releasable groups of Group II both R⁸ and R⁹ are hydrogen. Preferably, for releasable groups of Group II R⁷ is methyl or isopropyl. Optionally, R⁶, R⁷, R⁸, R⁹ comprised in said Group I, and II, are -(S^{P})ᵢ-C^{B}.

For all releasable groups according to Group I and Group II Y^{C1} is selected from the group consisting of -O-, -S-, and -NR⁶-, preferably -NR⁶-. For all linkers according to Group I, and Group II, Y^{C2} is selected from the group consisting of O and S, preferably O.

Releasable groups according to Group III are

| | |
|---|---|
| 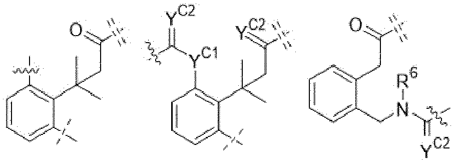 | 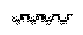 indicates bond to -O- on the allylic position of the trans-cyclooctene |
| | --- indicates optional bond to -(S^{P})ᵢ-C^{B} |
| | 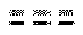 indicates bond to C^{A} |

, wherein the wiggly line may also indicate a bond to -S- on the allylic position of the trans-cyclooctene.

Releasable groups according to Group IV are

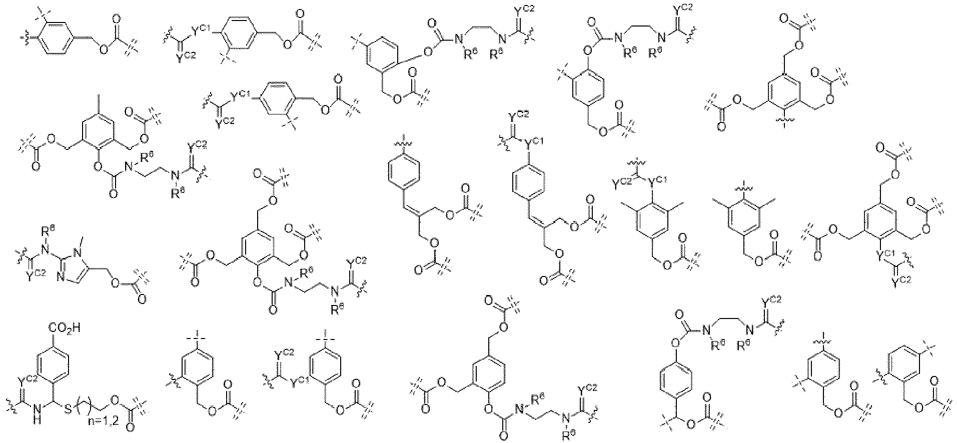

| | |
|---|---|
| 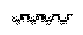 indicates bond to -O- on the allylic position of the trans-cyclooctene | 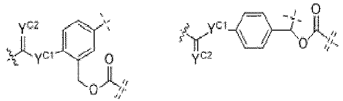 |
| - - - indicates optional bond to -(S^{P})ᵢ-C^{B} | |
| 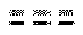 indicates bond to C^{A} | |

, wherein the wiggly line may also indicate a bond to -S- on the allylic position of the trans-cyclooctene.

Preferably, R⁶, R⁷, R⁸, R⁹ are according to RG1 or any preferred embodiment thereof. Preferably, R⁶, R⁷, R⁸, R⁹ used in this Section are not substituted. Most preferably, R⁶, R⁷, R⁸, R⁹ used in this Section are hydrogen.

### Compositions of the invention

The invention also pertains to a composition comprising a compound according to the invention, or the salt, hydrate, or solvate thereof. Preferably, the composition is a pharmaceutical composition. Preferably, the composition of the invention further comprises a pharmaceutically acceptable carrier. It is also preferred that if a salt of a compound of the invention is included in the composition of the invention, a pharmaceutically acceptable salt is used.

### Combinations of the invention

The invention also relates to a combination of (A1) a compound according to the invention, or the salt, hydrate, or solvate thereof; and/or (A2) a composition according to the invention; with (B) a diene or a salt, solvate, or hydrate thereof. Preferably the diene is a tetrazine.

Preferably, the combination of the invention is a kit. More preferably, the combination of the invention is a kit wherein (A1) and/or (A2) is/are physically separated from (B).

### Non-therapeutic methods using and uses for using compounds of the invention

The invention pertains to a non-therapeutic method and a non-therapeutic use. Preferably, the dienophile used therein is as described in relation to the combination of the invention.

For the non-therapeutic method of the invention it is preferred that the compound of the invention (viz. (ia)), the conjugate of the invention (viz. (iia)), and/or the composition of the invention (viz. (iiia)), and the diene are further contacted with a solvent. The skilled person is aware of suitable solvents for a reaction between a TCO and a tetrazine. Preferably, the solvent comprises water, and more preferably the solvent is water.

For the non-therapeutic use, the click reaction is preferably a bioorthogonal click reaction. Preferably, the click reaction is performed *in vitro,* although non-therapeutic reactions *in vivo* can be carried out as well.

### Definitions

The present invention is herein described with respect to particular embodiments, but the invention is not limited thereto but only by the claims. Where an indefinite or definite article is used when referring to a singular noun e.g. "a" or "an", "the", this includes a plural of that noun unless something else is specifically stated.

The verb "to comprise", and its conjugations, as used in this description and in the claims is used in its non-limiting sense to mean that items following the word are included, but items not specifically mentioned are not excluded.

In addition, reference to an element by the indefinite article "a" or "an" does not exclude the possibility that more than one of the element is present, unless the context clearly requires that there is one and only one of the elements. The indefinite article "a" or "an" thus usually means "at least one".

Thus, the scope of the expression "a device comprising means A and B" should not be limited to devices consisting only of components A and B. It means that with respect to the present invention, the only relevant components of the device are A and B.

The compounds herein may occur in different tautomeric forms. The compounds according to the invention are meant to include all tautomeric forms, unless stated otherwise. When the structure of a compound is depicted as a specific tautomer, it is to be understood that the invention of the present application is not limited to that specific tautomer, unless stated otherwise.

The compounds herein may occur in different enantiomeric forms. The compounds according to the invention are meant to include all enantiomeric forms, unless stated otherwise. When the structure of a compound is depicted as a specific enantiomer, it is to be understood that the invention of the present application is not limited to that specific enantiomer, unless stated otherwise.

Unless stated otherwise, the compounds of the invention and/or groups thereof may be protonated or deprotonated. It will be understood that it is possible that a compound may bear multiple charges which may be of opposite sign. For example, in a compound containing an amine and a carboxylic acid, the amine may be protonated while simultaneously the carboxylic acid is deprotonated.

In several formulae, groups or substituents are indicated with reference to letters such as "A", "B", "X", "Y", and various (numbered) "R" groups. In addition, the number of repeating units may be referred to with a letter, e.g. n in -(CH₂)ₙ-. The definitions of these letters are to be read with reference to each formula, i.e. in different formulae these letters, each independently, can have different meanings unless indicated otherwise.

Herein, reference is made to "alkyl", and the like. The number of carbon atoms that these groups have, excluding the carbon atoms comprised in any optional substituents as defined below, can be indicated by a designation preceding such terms (e.g. "C₁-C₈ alkyl" means that said alkyl may have from 1 to 8 carbon atoms). For the avoidance of doubt, a butyl group substituted with a -OCH₃ group is designated as a C₄ alkyl, because the carbon atom in the substituent is not included in the carbon count.

As used herein, alkyl groups are unsubstituted and have the general formula CₙH₂ₙ₊₁ and may be linear or branched. Examples of alkyl groups include methyl, ethyl, propyl, 2-propyl, t-butyl, 1-hexyl, 1-dodecyl, etc. Thus, C₁₋₃ alkyl groups are methyl, ethyl, 1-propyl, and 2-propyl.

The term "salt thereof' means a compound formed when an acidic proton, typically a proton of an acid, is replaced by a cation, such as a metal cation or an organic cation and the like. The term "salt thereof' also means a compound formed when an amine is protonated. Where applicable, the salt is a pharmaceutically acceptable salt, although this is not required for salts that are not intended for administration to a patient. For example, in a salt of a compound the compound may be protonated by an inorganic or organic acid to form a cation, with the conjugate base of the inorganic or organic acid as the anionic component of the salt.

The term "pharmaceutically accepted salt" means a salt that is acceptable for administration to a patient, such as a mammal (salts with counter-ions having acceptable mammalian safety for a given dosage regime). Such salts may be derived from pharmaceutically acceptable inorganic or organic bases and from pharmaceutically acceptable inorganic or organic acids.

"Pharmaceutically acceptable salt" refers to pharmaceutically acceptable salts of a compound, which salts are derived from a variety of organic and inorganic counter ions known in the art and include, for example, sodium, potassium, calcium, magnesium, ammonium, tetraalkylammonium, etc., and when the molecule contains a basic functionality, salts of organic or inorganic acids, such as hydrochloride, hydrobromide, formate, tartrate, besylate, mesylate, acetate, maleate, oxalate, etc.

It will be understood that herein, the terms "moiety" and "group" are used interchangeably when referring to a part of a molecule.

It will be understood that when a heteroatom is denoted as -X(R')₂-, wherein X is the heteroatom and R' is a certain moiety, then this denotes that two moieties R' are attached to the heteroatom.

It will be understood that when a group is denoted as, for example,
-((R₅₁)₂-R₅₂)₂- or a similar notation, in which R₅₁ and R₅₂ are certain moieties, then this denotes that first, it should be written as -R₅₁-R₅₁-R₅₂-R₅₁-R₅₁-R₅₂- before the individual R₅₁ and R₅₂ moieties are selected, rather than first selecting moieties R₅₁ and R₅₂ and then writing out the formula.

### Radical groups (RG)

### Radical Group 1: terminal groups

For Radical Group 1 (RG1), the radical is selected from the group consisting of -H, - Cl, -F, -Br, -I, -OH, -NH₂, -COOH, -CONH₂, -CN, -N₃, -NCS, -SCN, -SO₃H, -PO₃H -PO₄H₂, -NO₂, -CF₃, -CF₂H, -CFH₂, =O, =NH, -SH, -SO₂H, -(S^{P})ᵢ-C^{B}, (hetero)alkyl, (hetero)alkenyl, (hetero)alkynyl, (hetero)cycloalkyl, (hetero)cycloalkenyl, (hetero)cycloalkynyl, (hetero)aryl, and combinations thereof. Herein, S^{P} is a spacer as defined herein, C^{B} is Construct B as defined herein, and i is an integer in a range of from 0 to 4, preferably i is 0 or 1.

For RG1, "combinations thereof' in particular refers to (hetero)alkylcycloalkyl, (hetero)alkylcycloalkenyl, (hetero)alkylcycloalkynyl, (hetero)cycloalkylalkyl, (hetero)cycloalkenylalkyl, (hetero)cycloalkynylalkyl, (hetero)alkenylcycloalkyl, (hetero)alkenylcycloalkenyl, (hetero)alkenylcycloalkynyl, (hetero)cycloalkylalkenyl, (hetero)cycloalkenylalkenyl, (hetero)cycloalkynylalkenyl, (hetero)alkynylcycloalkyl, (hetero)alkynylcycloalkenyl, (hetero)alkynylcycloalkynyl, (hetero)cycloalkylalkynyl, (hetero)cycloalkenylalkynyl, (hetero)cycloalkynylalkynyl, (hetero)arylalkyl, (hetero)arylalkenyl, (hetero)arylalkynyl, alkyl(hetero)aryl, alkenyl(hetero)aryl, alkynyl(hetero)aryl, cycloalkyl(hetero)aryl, cycloalkenyl(hetero)aryl, cycloalkynyl(hetero)aryl, (hetero)arylcycloalkyl, (hetero)arylcycloalkenyl, and (hetero)arylcycloalkynyl. In addition, "combinations thereof' in relation to RG1 also refers to *e.g.* an alkyl group substituted with one or more -Cl and/or -OH groups. As such, RG1 also comprises radicals such as -NH-CH₂-COOH (a glycine residue), which is a combination of a heteroalkyl and -COOH.

Preferably, for RG1 the radical is selected from the group RG1a consisting of -H, -Cl, -F, -Br, -I, -OH, -NH₂, -COOH, -CONH₂, -SO₃H, -PO₃H -PO₄H₂, -NO₂, -CF₃, =O, =NH, -SH, -(S^{P})ᵢ-C^{B}, C₁-C₂₄ (hetero)alkyl, C₂-C₂₄ (hetero)alkenyl, C₂-C₂₄ (hetero)alkynyl, C₃-C₂₄ cycloalkyl, C₂-C₂₄ heterocycloalkyl, C₅-C₂₄ cycloalkenyl, C₃-C₂₄ heterocycloalkenyl, C₇-C₂₄ cycloalkynyl, C₅-C₂₄ (hetero)cycloalkynyl, C₆-C₂₄ aryl, C₂-C₂₄ heteroaryl, and combinations thereof.

More preferably, for RG1 the radical is selected from the group RG1b consisting of - H, -Cl, -F, -Br, -I, -OH, -NH₂, -COOH, -CONH₂, -SO₃H, -PO₃H -PO₄H₂, -NO₂, -CF₃, =O, =NH, -SH, -(S^{P})ᵢ-C^{B}, C₁-C₁₂ (hetero)alkyl, C₂-C₁₂ (hetero)alkenyl, C₂-C₁₂ (hetero)alkynyl, C₃-C₁₂ cycloalkyl, C₂-C₁₂ heterocycloalkyl, C₅-C₁₂ cycloalkenyl, C₃-C₁₂ heterocycloalkenyl, C₇-C₁₂ cycloalkynyl, C₅-C₁₂ (hetero)cycloalkynyl, C₆-C₁₂ aryl, C₂-C₁₂ heteroaryl, and combinations thereof.

Even more preferably, for RG1 the radical is selected from the group RG1c consisting of -H, -Cl, -F, -Br, -I, -OH, -NH₂, -COOH, -CONH₂, -SO₃H, -PO₃H -PO₄H₂, -NOz, -CF₃, =O, =NH, -SH, -(S^{P})ᵢ-C^{B}, C₁-C₈ (hetero)alkyl, C₂-C₈ (hetero)alkenyl, C₂-C₈ (hetero)alkynyl, C₃-C₈ cycloalkyl, C₂-C₈ heterocycloalkyl, C₅-C₈ cycloalkenyl, C₃-C₈ heterocycloalkenyl, C₇-C₈ cycloalkynyl, C₅-C₈ (hetero)cycloalkynyl, C₆-C₈ aryl, C₂-C₈ heteroaryl, and combinations thereof.

More preferably still, for RG1 the radical is selected from the group RG1d consisting of -H, -Cl, -F, -Br, -I, -OH, -NH₂, -COOH, -CONH₂, -SO₃H, -PO₃H -PO₄H₂, -NOz, -CF₃, =O, =NH, -SH, -(S^{P})ᵢ-C^{B}, C₁-C₆ (hetero)alkyl, C₂-C₆ (hetero)alkenyl, C₂-C₆ (hetero)alkynyl, C₃-C₆ cycloalkyl, C₂-C₆ heterocycloalkyl, C₅-C₇ cycloalkenyl, C₃-C₅ heterocycloalkenyl, C₈ cycloalkynyl, C₆-C₇ (hetero)cycloalkynyl, phenyl, C₃-C₅ heteroaryl, and combinations thereof.

Most preferably, for RG1 the radical is selected from the group RG1e consisting of -H, -Cl, -F, -Br, -I, -OH, -NH₂, -COOH, -CONH₂, -SO₃H, -PO₃H -PO₄H₂, -NO₂, -CF₃, =O, =NH, -SH, -(S^{P})ᵢ-C^{B}, C₁-C₃ (hetero)alkyl, C₃-C₆ cycloalkyl, C₂-C₅ heterocycloalkyl, phenyl, C₄-C₅ heteroaryl, and combinations thereof.

In some embodiments, for RG1 the radical is a conjugation moiety, which is a chemical group that can be used for binding, conjugation or coupling of a Construct, such as Construct-B, or a Spacer, or another molecule or construct of interest. The person skilled in the art is aware of the myriad of strategies that are available for the chemoselective or -unselective or enzymatic coupling or conjugation of one molecule or construct to another.

In some embodiments, RG1 is a moiety that allows conjugation to a protein comprising natural and/or non-natural amino acids. Moieties suitable for conjugation are known to the skilled person. Conjugation strategies are for example found in [O. Boutureira, G.J.L. Bernardes, Chem. Rev., 2015, 115, 2174-2195].

If RG1 is a conjugation moiety, it is preferably selected from the group RG1f consisting of N-maleimidyl, halogenated N-alkylamido, sulfonyloxy N-alkylamido, vinyl sulfone, (activated) carboxylic acids, benzenesulfonyl halides, ester, carbonate, sulfonyl halide, thiol or derivatives thereof, C₂₋₆ alkenyl, C₂₋₆ alkynyl, C₇₋₁₈ cycloalkynyl, C₅₋₁₈ heterocycloalkynyl, bicyclo[6.1.0]non-4-yn-9-yl], C₃₋₁₂ cycloalkenyl, azido, phosphine, nitrile oxide, nitrone, nitrile imine, isonitrile, diazo, ketone, (O-alkyl)hydroxylamino, hydrazine, halogenated N-maleimidyl, aryloxymaleimides, dithiophenolmaleimides, bromo- and dibromopyridazinediones, 2,5-dibromohexanediamide, alkynone, 3-arylpropiolonitrile, 1,1-bis(sulfonylmethyl)-methylcarbonyl or elimination derivatives thereof, carbonyl halide, allenamide, 1,2-quinone, isothiocyanate, isocyanate, aldehyde, triazine, squaric acids, 2-imino-2-methoxyethyl, (oxa)norbornene, (imino)sydnones, methylsulfonyl phenyloxadiazole, aminooxy, 2-amino benzamidoxime, ethynylphosphonamidates, reactive in the Pictet-Spengler ligation and hydrazine- Pictet-Spengler (HIPS) ligation, DNA intercalators, tetrazine, trans-cyclooctene, and photocrosslinkers. More preferably, RG1f is N-maleimidyl.

In other embodiments RG1f is selected from the group consisting of hydroxyl, amine, halogens, vinyl pyridine, disulfide, pyridyl disulfide, sulfonyloxy, mercaptoacetamide, anhydride, sulfonylated hydroxyacetamido, sulfonyl chlorides, thiosemicarbazone, hydrazine carboxylate, and arylhydrazide. In yet other embodiments RG1f is a group that can be connected to another group by means of an enzyme, for example sortase or Tubulin tyrosine ligase.

### Radical Group 2: connecting groups

For Radical Group 2 (RG2), the radical is selected from the group consisting of (hetero)alkylene, (hetero)alkenylene, (hetero)alkynylene, (hetero)cycloalkylene, (hetero)cycloalkenylene, (hetero)cycloalkynylene, (hetero)arylene, amino acid, peptide, protein, polymer, oligonucleotide, nucleotide, carbohydrate, RG2a, RG2b, RG2c, and combinations thereof.

The radicals from RG2 are optionally attached to one or more radicals according to RG1. Thus, RG2 also covers *e.g.* -NH-CH(CHzOH)-C(O)- (*i.e.* a serine residue), which is a heteroalkylene attached to -OH and =O.

For RG2, "combinations thereof' in particular, but not exclusively, refers to alkyl(hetero)arylene, (hetero)arylalkylene, (hetero)arylalkenylene, (hetero)arylalkynylene, alkenyl(hetero)arylene, and alkynyl(hetero)arylene.

Preferably, for RG2 the radical is selected from the group consisting of C₁-C₂₄ (hetero)alkylene, C₂-C₂₄ (hetero)alkenylene, C₂-C₂₄ (hetero)alkynylene, C₃-C₂₄ cycloalkylene, C₂-C₂₄ heterocycloalkylene, C₅-C₂₄ cycloalkenylene, C₃-C₂₄ heterocycloalkenylene, C₇-C₂₄ cycloalkynylene, C₅-C₂₄ (hetero)cycloalkynylene, C₆-C₂₄ arylene, C₂-C₂₄ heteroarylene, amino acid, peptide, protein, polymer, oligonucleotide, nucleotide, carbohydrate, RG2a, RG2b, RG2c, and combinations thereof.

More preferably, for RG2 the radical is selected from the group consisting of C₁-C₁₂ (hetero)alkylene, C₂-C₁₂ (hetero)alkenylene, C₂-C₁₂ (hetero)alkynylene, C₃-C₁₂ cycloalkylene, C₂-C₁₂ heterocycloalkylene, C₅-C₁₂ cycloalkenylene, C₃-C₁₂ heterocycloalkenylene, C₇-C₁₂ cycloalkynylene, C₅-C₁₂ (hetero)cycloalkynylene, C₆-C₁₂ arylene, C₂-C₁₂ heteroarylene, amino acid, peptide, protein, polymer, oligonucleotide, nucleotide, carbohydrate, RG2a, RG2b, RG2c, and combinations thereof.

Even more preferably, for RG2 the radical is selected from the group consisting of C₁-Ca (hetero)alkylene, C₂-C₈ (hetero)alkenylene, C₂-C₈ (hetero)alkynylene, C₃-C₈ cycloalkylene, C₂-C₈ heterocycloalkylene, C₅-C₈ cycloalkenylene, C₃-C₈ heterocycloalkenylene, C₇-C₈ cycloalkynylene, C₅-C₈ (hetero)cycloalkynylene, C₆-C₈ arylene, C₂-C₈ heteroarylene, amino acid, peptide, protein, polymer, oligonucleotide, nucleotide, carbohydrate, RG2a, RG2b, RG2c, and combinations thereof.

More preferably still, for RG2 the radical is selected from the group consisting of C₁-C₆ (hetero)alkylene, C₂-C₆ (hetero)alkenylene, C₂-C₆ (hetero)alkynylene, C₃-C₆ cycloalkylene, C₂-C₆ heterocycloalkylene, C₅-C₇ cycloalkenylene, C₃-C₅ heterocycloalkenylene, C₈ cycloalkynylene, C₆-C₇ (hetero)cycloalkynylene, phenylene, C₃-C₅ heteroarylene, amino acid, peptide, protein, polymer, oligonucleotide, nucleotide, carbohydrate, RG2a, RG2b, RG2c, and combinations thereof.

Even more preferably still, for RG2 the radical is selected from the group consisting of C₁-C₃ (hetero)alkylene, C₃-C₆ cycloalkylene, C₂-C₅ heterocycloalkylene, phenylene, C₄-C₅ heteroarylene, amino acid, peptide, protein, polymer, oligonucleotide, nucleotide, carbohydrate, RG2a, RG2b, RG2c, and combinations thereof.

RG2a is selected from the group consisting of -O-, -S-, -SS-, -NR₄-, -N=N-, -C(O)-, - C(O)NR₄-, -OC(O)-, -C(O)O-, -OC(O)O-, -OC(O)NR₄-, -NR₄C(O)-, -NR₄C(O)O-, - NR₄C(O)NR₄-, -SC(O)-, -C(O)S-, -SC(O)O-, -OC(O)S-, -SC(O)NR₄-, -NR₄C(O)S-, -S(O)-, - S(O)₂-, -OS(O)₂-, -S(O₂)O-, -OS(O)₂O-, -OS(O)₂NR₄-, -NR₄S(O)₂O-, -C(O)NR₄S(O)₂NR₄-, - OC(O)NR₄S(O)₂NR₄-, -OS(O)-, -OS(O)O-, -OS(O)NR₄-, -ONR₄C(O)-, -ONR₄C(O)O-, - ONR₄C(O)NR₄-, -NR₄OC(O)-, -NR₄OC(O)O-, -NR₄OC(O)NR₄-, -ONR₄C(S)-, -ONR₄C(S)O-, -ONR₄C(S)NR₄-, -NR₄OC(S)-, -NR₄OC(S)O-, -NR₄OC(S)NR₄-, -OC(S)-, -C(S)O-, - OC(S)O-, -OC(S)NR₄-, -NR₄C(S)-, -NR₄C(S)O-, -SS(O)₂-, -S(O)₂S-, -OS(O₂)S-, -SS(O)₂O-, - NR₄OS(O)-, -NR₄OS(O)O-, -NR₄OS(O)NR₄-, -NR₄OS(O)₂-, -NR₄OS(O)₂O-, - NR₄OS(O)₂NR₄-, -ONR₄S(O)-, -ONR₄S(O)O-, -ONR₄S(O)NR₄-, -ONR₄S(O)₂O-, - ONR₄S(O)₂NR₄-, -ONR₄S(O)₂-, -OP(O)(R₄)₂-, -SP(O)(R₄)₂-, and -NR₄P(O)(R₄)₂-. Herein, R₄ is according to RG1, preferably R₄ is hydrogen or methyl, more preferably R₄ is hydrogen.

Preferably, RG2a is selected from the group consisting of -O-, -S-, -SS-, -NR₄-, -N=N-, -C(O)-, -C(O)NR₄-, -OC(O)-, -C(O)O-, -OC(O)NR₄-, -NR₄C(O)-, -NR₄C(O)O-, - NR₄C(O)NR₄-, -SC(O)-, -C(O)S-, -SC(O)O-, -OC(O)S-, -SC(O)NR₄-, -NR₄C(O)S-, -S(O)-, - S(O)₂-, -C(O)NR₄S(O)₂NR₄-, -OC(O)NR₄S(O)₂NR₄-, -OC(S)-, -C(S)O-, -OC(S)O-, - OC(S)NR₄-, -NR₄C(S)-, -NR₄C(S)O-, and -SS(O)₂-.

More preferably, for RG2 the radical is RG2b or RG2c, most preferably RG2b.

RG2b is selected from the group consisting of

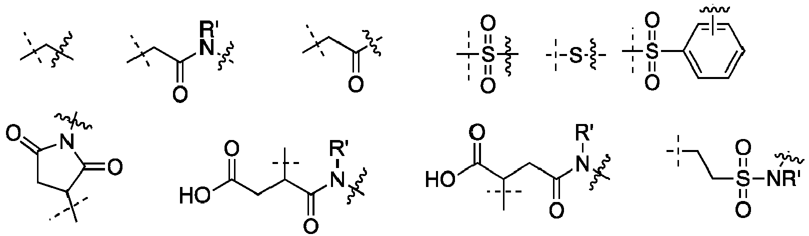

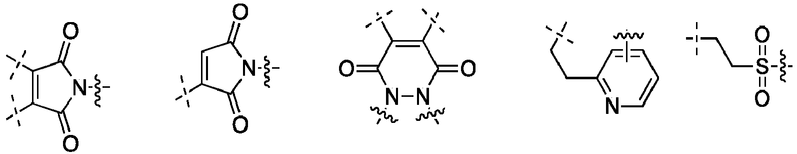

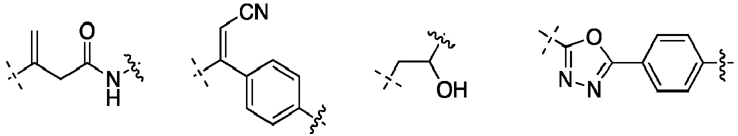

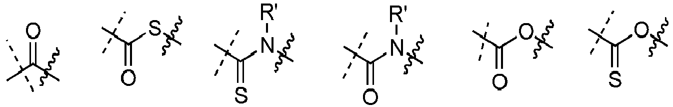

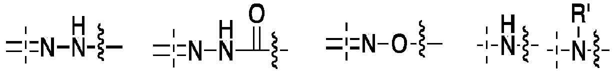

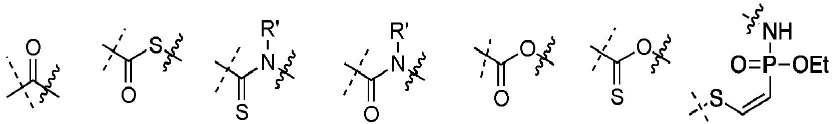

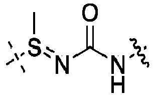

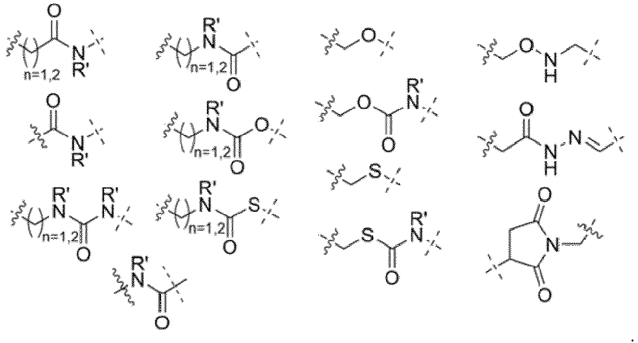

Therein, R' is a radical according to RG1, preferably R' is hydrogen or C₁₋₃ alkyl. The dashed and wiggly lines denote bonds to the other parts of the molecule.

RG2c is selected from the group consisting of

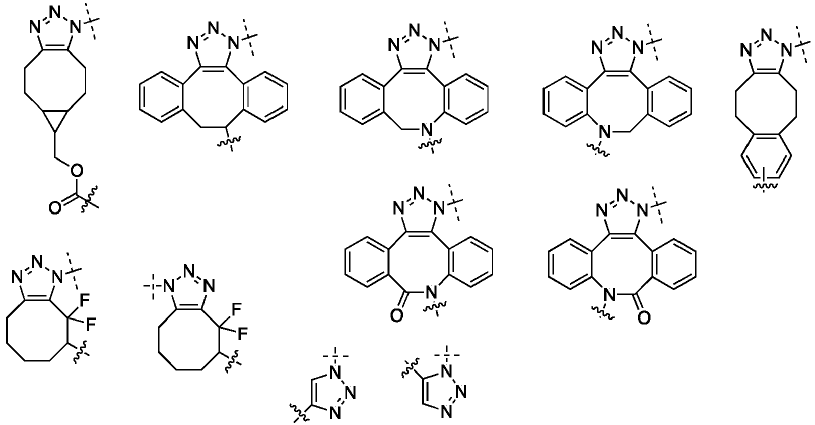

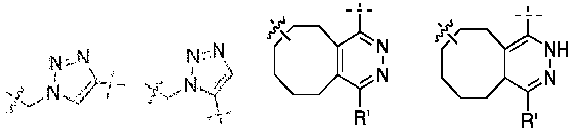

Therein, R' is a radical according to RG1, preferably R' is hydrogen or C₁₋₃ alkyl. The dashed and wiggly lines denote bonds to the other parts of the molecule.

### Examples

### Example 1: Synthesis of compounds 1 and 2

Intermediate compound 3 was obtained in three steps with an overall yield of 29% using commercially available starting materials:

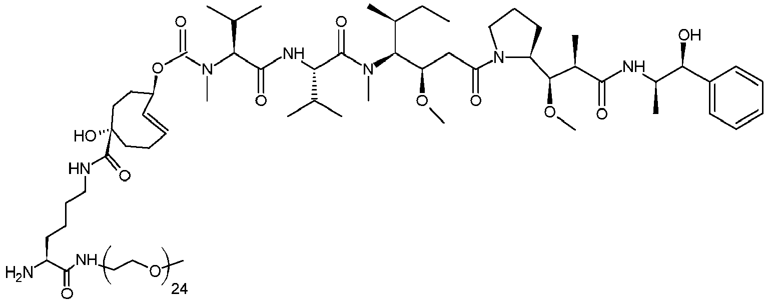

### Compound 3.

Compounds **4** and **5** were synthesized by coupling compound **3** to maleimide-PEG4-COOH or maleimide-C5-COOH, respectively, using conventional coupling reagents. This afforded the desired products, viz. compounds **4** and **5**, in high yield.

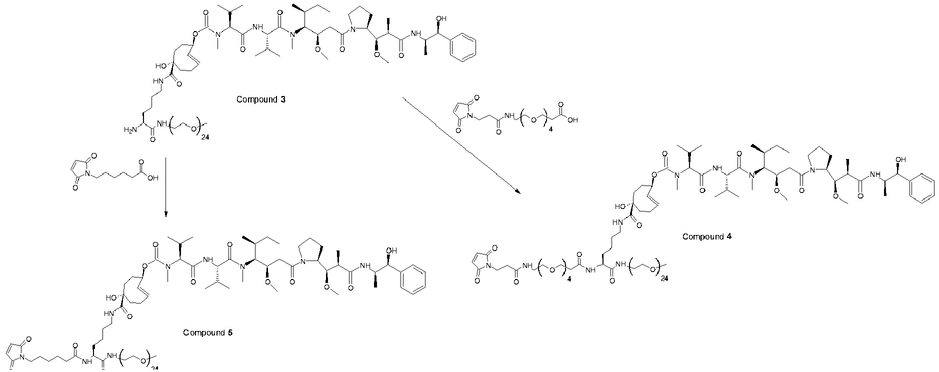

Compound **4** or **5**, respectively, was conjugated to diabody AVP0458 to afford compound **1** or **2** following the optimized procedure by Rossin et al., Nature Communications (2018)9:1484.

Briefly, 10 mg AVP0458 was reacted with 6 mM DTT for 3 h at room temperature on a roller bench, followed by purification via PD-10 pre-equilibrated with 0.1 M phosphate buffer pH 6.8, containing 2 mM ETDA (PB-EDTA buffer). The purified diabody solution was then split in two aliquots which were then added with 30 eq of compound **4** or compound **5**, respectively, dissolved in dry DMSO at 10 mM concentration. The two reaction mixtures were incubated for 1h at room temperature on a roller bench followed by overnight incubation at +4°C. The two resulting conjugates ADCs (compound **1**, conjugate of AVP0458 and compound **4**; and compound **2**, conjugate of AVP0458 and compound **5**) were then purified from the crude mixtures by SEC (Superdex75 10/300 column eluted with PBS at 0.8 mL/min) followed by concentration via Amicon Ultra-4 (30 kDa MW cut-off). UV measurements on the final solutions showed 75-80% recovery of diabody. SDS-PAGE analysis of the two ADC solutions showed the presence of one species with the expected increase in MW with respect to that of the monomer in AVP0458. Further analysis using mass spectrometry showed a complete reaction between the four cysteine residues in AVP0458 with the respective TCOs, confirming the production of two conjugates with a DAR of 4.

Compound **2** has the following structure:

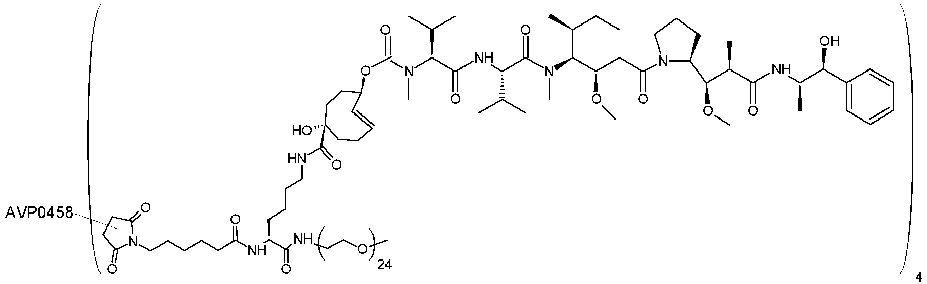

### Example 2: in vivo blood clearance of compounds 1 and 2 in mice

Two groups of tumor free mice (n=3) were injected 125I-labeled compound **1** or compound **2** (5 mg/kg) and blood samples (ca 50 µL) were withdrawn from the vena saphena at various times up to 72h post injection. After gamma-counting, plasma isolated from blood was reacted ex vivo with an excess of tetrazine **6** for at least 3h at 37°C.

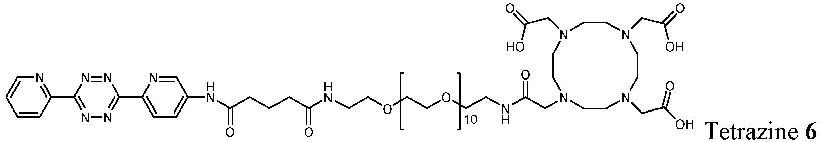

Then, the samples were analyzed by SEC on a Superdex75 10/300 column eluted with PBS at 0.8 mL/min. The eluates were collected in 1 ml fractions which were then measured by gamma-counting using a dual-isotope protocol with crossover correction. From this, the amount of compound **1** or **2** in the blood of the mice 48 hours after injection could be determined, as well as the respective half-lives in blood for said compounds. These results are shown in Table 1:

**Table 1. Blood clearance in mice of compounds 1 and 2.**

| | **Compound left in blood of mice 48h post-injection** (in %ID/g) | **Half-lives of compounds in blood of mice** (in hours) |
|---|---|---|
| Compound **1** (reference) | 1.14 ± 0.15 | 4.22 |
| Compound **2** | 0.82 ± 0.06 | 4.20 |

Thus, compound **2** advantageously and surprisingly has a faster clearance rate than compound **1**. Furthermore, it was observed that both compounds **1** and **2** showed high *in vivo* TCO stability.

### Example 3: in vivo tumor and off-target binding of compounds 1 and 2 in mice

Two groups of mice (n=5) bearing LS174T xenografts were injected compound **1** (reference) or compound **2** (2 mg/kg) followed 49h later by 111In-labeled tetrazine **6** (10 eq with respect to ADC). Three hours post tetrazine **6** injection, the mice were euthanized and blood, tumors and other tissues were harvested, weighed and counted (together with standards) in a gamma counter with dual isotope protocol with crossover correction. The results are shown in Tables 2 and 3.

**Table 2. Biodistribution of compound 1 (reference) and compound 2 in tumor-bearing mice.**

| | **Amount of compound 1** (reference) (in %ID/g) | **Amount of compound 2** (invention) (in %ID/g) |
|---|---|---|
| **Tumour** | 18.42 ± 5.14 | 23.11 ± 6.21^{a} |
| **Blood** | 0.77 ± 0.12 | 0.52 ± 0.10 |
| **Heart** | 0.22 ± 0.04 | 0.15 ± 0.03 |
| **Lung** | 0.59 ± 0.06 | 0.44 ± 0.06 |
| **Liver** | 0.43 ± 0.20 | 0.38 ± 0.22 |
| **Spleen** | 0.27 ± 0.08 | 0.21 ± 0.07 |
| **Kidney** | 0.60 ± 0.08 | 0.57 ± 0.14 |
| **Muscle** | 0.08 ± 0.02 | 0.06 ± 0.01 |
| **Bone** | 0.11 ± 0.01 | 0.10 ± 0.02 |

| | | |
|---|---|---|
| ^{a}n = 4 (one mouse did not develop a tumour). | | |

**Table 3. Biodistribution of compound 1 (reference) and compound 2 in tumor-bearing mice.**

| | **Amount of compound 1** (reference) (in %ID/organ) | **Amount of compound 2** (invention) (in %ID/organ) |
|---|---|---|
| **Stomach full** | 0.04 ± 0.01 | 0.03 ± 0.01 |
| **Small intestine full** | 0.27 ± 0.03 | 0.20 ± 0.04 |
| **Large intestine full** | 0.17 ± 0.03 | 0.16 ± 0.04 |
| **Thyroid** | 0.48 ± 0.11 | 0.42 ± 0.07 |

From Tables 2 and 3 it is clear that compound **2** shows a higher uptake in tumour and a lower off-target uptake than reference compound **1**. These results are highly advantageous, since the higher the tumour/off target ratio, the lower the extent of unwanted side-effects are usually observed.

### Example 4: further in vitro and in vivo properties of compound 2

Other properties of compound **2** were tested as well:
1. *In vitro* metabolism studies were carried out using compound **1** or **2** in the presence or absence of acidified human liver S9 fraction for up to 24 hours. From these studies, it was shown that compound **2** has a better metabolism profile than compound **1**.
2. An *in vitro* reaction of compound **2** with a standard tetrazine yielded quantitative MMAE release after 24 hours of incubation.
3. At most 0.8% MMAE release was detected after 6 days of incubating compound **2** in mouse plasma in the absence of a tetrazine or any other trigger.

## Claims

1. A compound or a salt, hydrate, or solvate thereof; wherein said compound has a structure according to Formula (1): 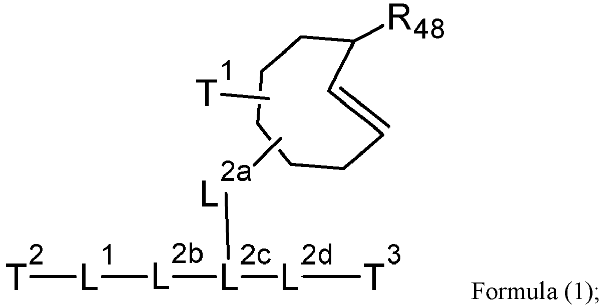 wherein
**L¹** is selected from the group consisting of linear or branched C₄-C₁₂ alkylene, C₃-C₈ (hetero)cycloalkylene, C₆-C₁₂ arylene, and C₄-C₁₁ heteroarylene;
**L^{2a}**, **L^{2b}**, and **L^{2d}** are each independently a linker;
**L^{2c}** is selected from the group consisting of C₁-C₈ (hetero)alkanetriyl, C₅-C₆ (hetero)arenetriyl. C₃-C₇ cycloalkanetriyl, and C₂-C₇ heterocycloalkanetriyl;
**T¹** is selected from the group consisting of -OT^{1A}, hydrogen, C₂-C₆ alkyl, C₆ aryl, C₄-C₅ heteroaryl, C₃-C₆ cycloalkyl, C₅-C₁₂ alkyl(hetero)aryl, C₅-C₁₂ (hetero)arylalkyl, C₄-C₁₂ alkylcycloalkyl, -N(T^{1A})₂, -ST^{1A}, -SO₃H, -C(O)T^{1A}, -C(O)OT^{1A}, -O-C(O)T^{1A} -C(O)N(T^{1A})₂, -N(T^{1A})₂-CO-T^{1A}, and -Si(T^{1A})₃;
each **T^{1A}** is independently selected from the group consisting of hydrogen, (hetero)alkyl, (hetero)alkenyl, (hetero)alkynyl, (hetero)aryl, and an amino acid residue;
**T²** is a bioconjugation moiety or a group -L³-C^{B}; wherein
**L³** is a residue of a bioconjugation moiety, and
**C^{B}** is selected from the group consisting of proteins, nucleic acids, peptides, carbohydrates, aptamers, lipids, small organic molecules, polymers, LNA, PNA, amino acids, peptoids, chelating moieties, fluorescent dyes, phosphorescent dyes, organic particles, gels, cells, and combinations thereof;
**T³** is a polymer; and
**R₄₈** is selected from the group consisting of -OH, -O-acetyl, -O-C₁₋₄ alkyl, halogen, active carbonate, and a releasable group; and
preferably **L¹** is linear or branched C₄-C₁₂ alkylene, more preferably **L¹** is linear or branched C₄-C₁₀ alkylene, and most preferably **L¹** is linear C₅-C₆ alkylene;
preferably L^{2a}, L^{2b}, and L^{2d} are each independently a linker containing at most twenty atoms; more preferably L^{2a}, L^{2b}, and L^{2d} are each independently selected from the group consisting of -C(O)NL^{2T}-, -NL^{2T}C(O)-, -O-, -S-, -NL^{2T}-, -N=N-, and -C(O)-; wherein L^{2T} is hydrogen or methyl, preferably L^{2T} is hydrogen;
preferably L^{2c} is C₁-C₈ (hetero)alkanetriyl, more preferably L^{2c} is C₁-C₈ alkanetriyl, and most preferably L^{2c} is C₄-C₆ alkanetriyl;
preferably T¹ is -OT^{1A}; and most preferably T¹ is -OH;
preferably T^{1A} is hydrogen or methyl, more preferably T^{1A} is hydrogen;
preferably T² is maleimidyl, N-hydroxysuccinimidyl, or -L³-C^{B};
preferably L³ is a residue of a maleimidyl moiety or a residue of an N-hydroxysuccinimidyl moiety;
preferably C^{B} is a protein, more preferably C^{B} is an antibody or a diabody, even more preferably C^{B} is a diabody, and most preferably C^{B} is AVP0458 according to SEQ ID NO: 1;
preferably T³ is a polymer comprising a polyethylene glycol moiety; and
preferably R₄₈ is a releasable group.

2. The compound according to claim 1, or a salt, hydrate, or solvate thereof; wherein said compound is according to Formula (2): 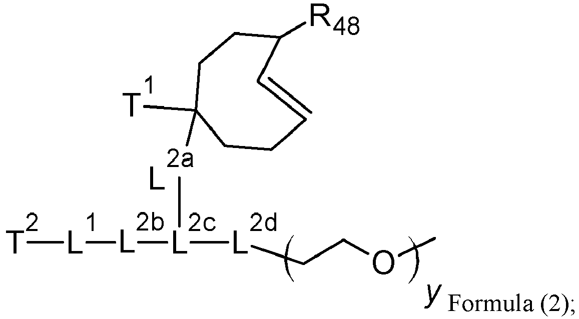 wherein y is an integer in a range of from 1 to 50;
preferably y is an integer in a range of from 10 to 40, more preferably in a range of from 12 to 37, even more preferably in a range of from 15 to 35, more preferably still in a range of from 20 to 30, and most preferably in a range of from 23 to 25.

3. The compound according to any one of the preceding claims, or a salt, hydrate, or solvate thereof; wherein said compound is according to Formula (3): 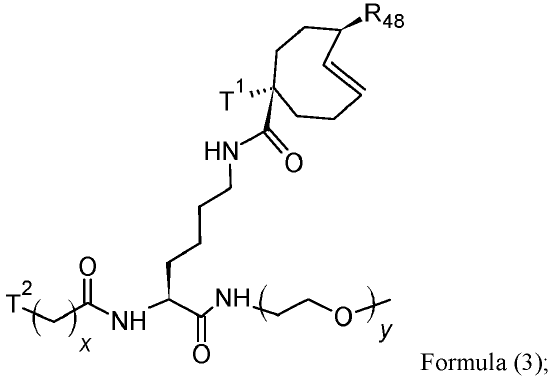 wherein
y is as defined in claim 2;
x is an integer in a range of from 4 to 12;
preferably x is an integer in a range of from 4 to 8, more preferably x is an integer in a range of from 4 to 6.

4. The compound according to any one of the preceding claims, or a salt, hydrate, or solvate thereof; wherein R₄₈ is a releasable group, and said releasable group is -O-CO-C^{A}; wherein C^{A} is a drug;
preferably the drug is linked to the moiety -O-CO- via a secondary or tertiary nitrogen atom that is part of the drug, forming a carbamate;
preferably the drug is monomethyl auristatin E (MMAE).

5. The compound according to any one of the preceding claims, or a salt, hydrate, or solvate thereof; wherein
T² is 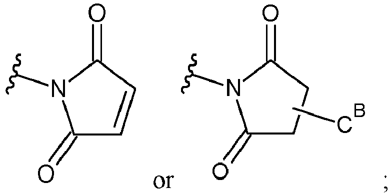 and
C^{B} is a protein;
preferably C^{B} is an antibody or a diabody, more preferably a diabody, and most
preferably AVP0458 according to SEQ ID NO: 1.

6. The compound according to any one of the preceding claims, or a salt, hydrate, or solvate thereof; wherein said compound is:

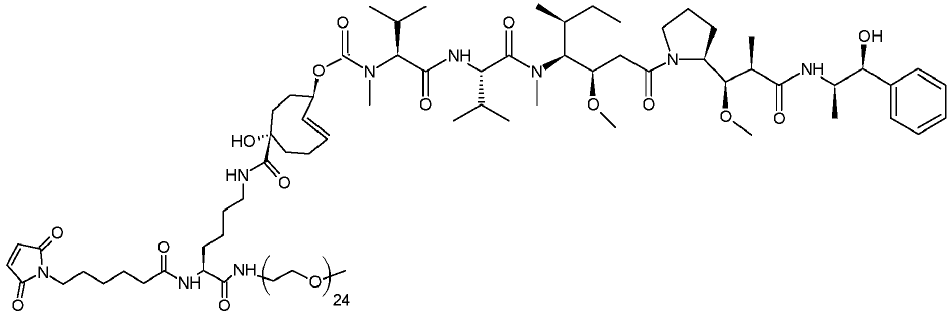

7. The compound according to any one of the preceding claims, or a salt, hydrate, or solvate thereof; wherein said compound is 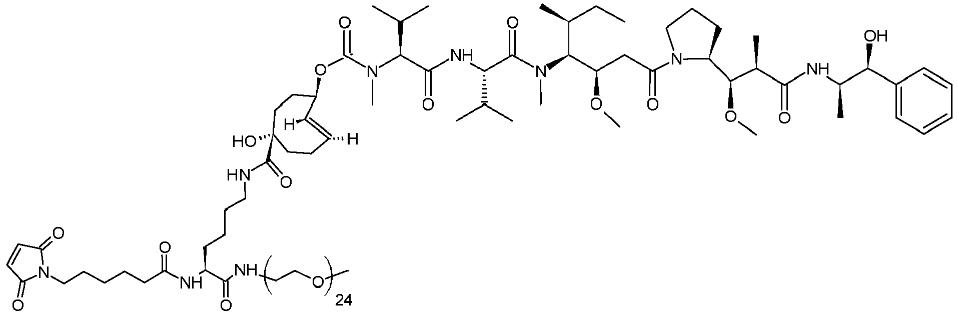 or

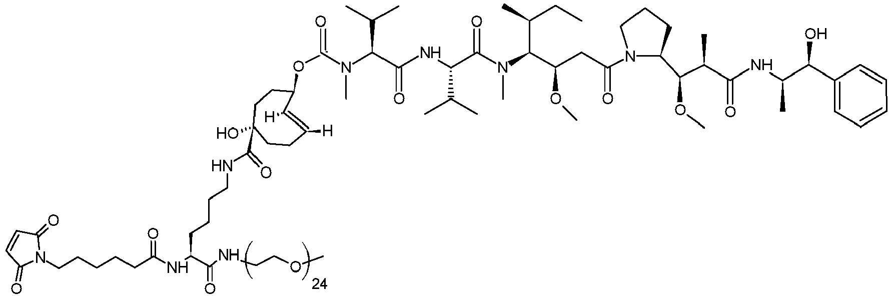

8. The compound according to any one of claims 1 to 5, or a salt, hydrate, or solvate thereof; wherein said compound is: 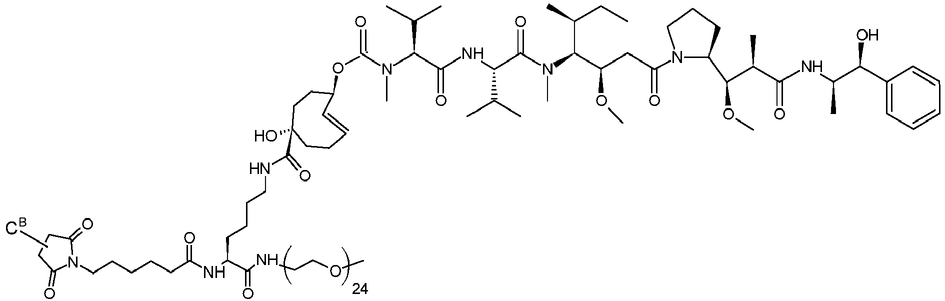 wherein C^{B} is AVP0458 according to SEQ ID NO: 1;
preferably C^{B} is linked to the maleimidyl group via a sulfur atom, preferably the sulfur atom is part of a cysteine.

9. The compound according to claim 8, or a salt, hydrate, or solvate thereof; wherein said compound is: 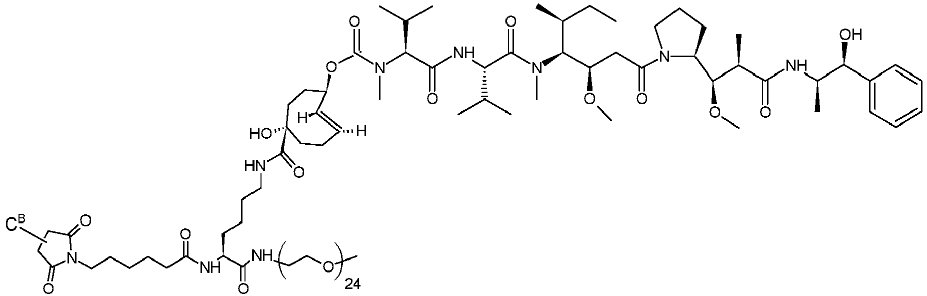 or

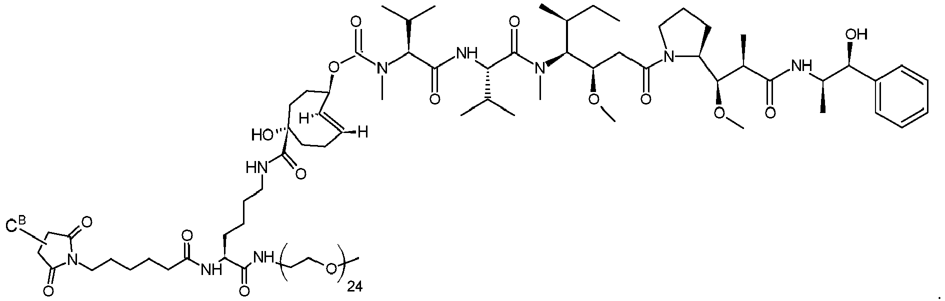

10. A compound or a salt, hydrate, or solvate thereof; wherein said compound comprises an eight-membered non-aromatic cyclic mono-alkenylene moiety, wherein said moiety comprises a non-vinylic carbon atom, wherein said non-vinylic carbon atom is substituted with at least one structure according to Formula (A): 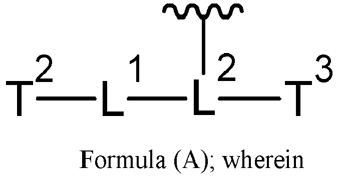 wherein
L¹ and L² are each independently a linker; and
T² and T³ are organic moieties.

11. A conjugate, or a salt, hydrate, or solvate thereof, wherein the conjugate comprises a protein conjugated to at least one compound according to Formula (1) as defined in any one of claims 1 to 9, wherein L¹, L^{2a}, L^{2b}, L^{2c}, L ^{2d}, T¹, T³, and R⁴⁸ are as defined in any one of claims 1 to 9, and wherein T² is a residue of a bioconjugation moiety, and said protein and said compound are conjugated via T²;
preferably the protein is a diabody or an antibody; more preferably the protein is a diabody; and most preferably the protein is AVP0458 according to SEQ ID NO: 1;
preferably the protein is conjugated to at most 12 of said compounds; more
preferably the protein is conjugated to at most 8 of said compounds, most
preferably the protein is conjugated to at most 4 of said compounds;
preferably said protein and said compound are conjugated via T² and a residue of a sulfhydryl of said protein, a residue of a hydroxyl of said protein, or a residue of an amine of said protein; more preferably said protein and said compound are conjugated via T² and a residue of a sulfhydryl of said protein;
preferably T² is a residue of a maleimidyl moiety or a residue of an N-hydroxysuccinimidyl moiety; more preferably T² is a residue of a maleimidyl moiety.

12. The conjugate according to claim 11, or a salt, hydrate, or solvate thereof, wherein the conjugate is 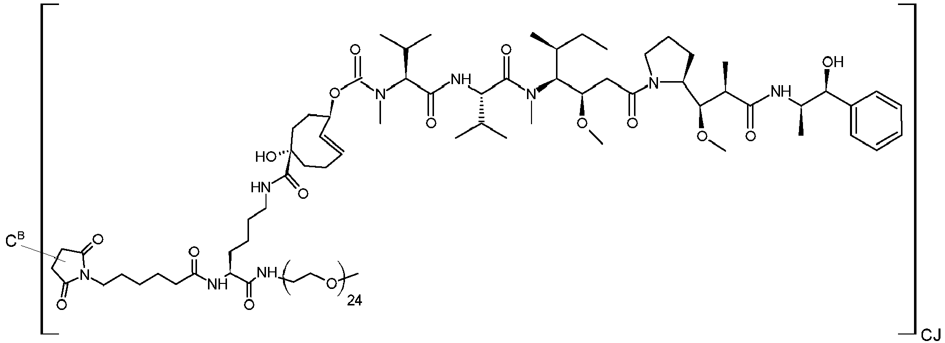
wherein CJ is in a range of from 1 to 12;
wherein C^{B} is AVP0458 according to SEQ ID NO: 1;
preferably CJ is of from 2 to 10, more preferably of from 2.5 to 8, even more
preferably of from 3 to 6, even more preferably still of from 3.5 to 4, and most
preferably about 4;
preferably C^{B} is linked to each maleimidyl group via a sulfur atom, preferably the sulfur atom is part of a cysteine.

13. The conjugate according to claim 12, or a salt, hydrate, or solvate thereof, wherein the conjugate is 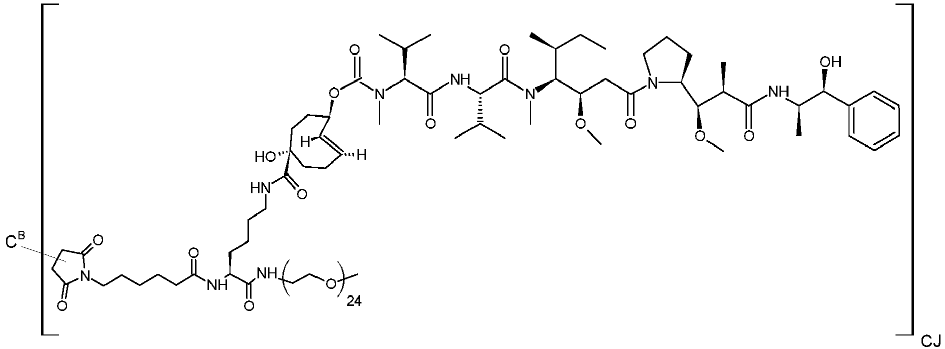 or

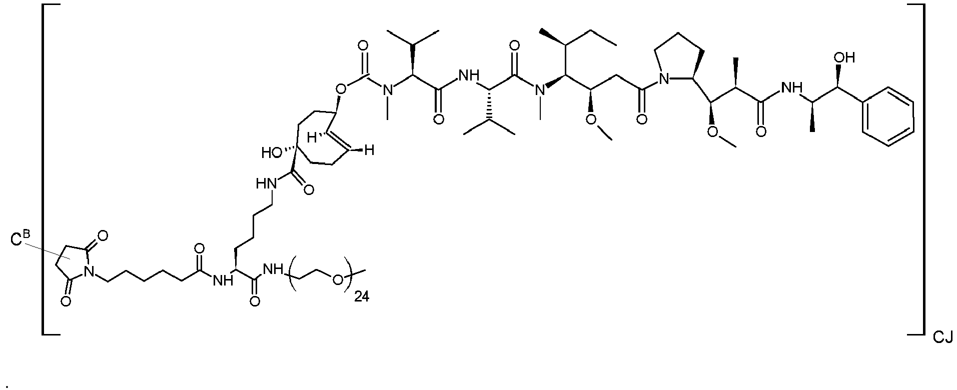

14. A composition comprising:
(a) a compound according to any one of claims 1 to 10, or the salt, hydrate, or solvate thereof; and/or
(b) the conjugate according to any one of claims 11 to 13, or the salt, hydrate, or solvate thereof;
preferably the composition is a pharmaceutical composition.

15. A composition according to claim 14, wherein said composition comprises:
(a) a compound according to any one of claims 1 to 10, or the salt, hydrate, or solvate thereof; and
(b) the enantiomer of said compound, or the salt, hydrate, or solvate thereof;
preferably said composition is a racemic mixture of (a) and (b).

16. A combination of
(A1) a compound according to any one of claims 1 to 10, or the salt, hydrate, or solvate thereof;
(A2) a conjugate according to any one of claims 11 to 13, or the salt, hydrate, or solvate thereof; and/or
(A3) a composition according to claim 14 or 15: with
(B) a diene or a salt, solvate, or hydrate thereof;
preferably the diene is a tetrazine.

17. The combination according to claim 16, wherein the diene is 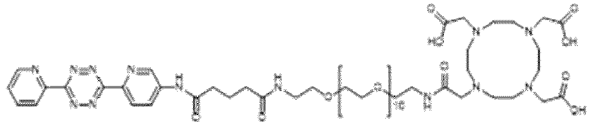 ; or a salt, hydrate, and/or solvate thereof.

18. The compound according to any one of claims 1 to 10, or the salt, hydrate, or solvate thereof; the conjugate according to any one of claims 11 to 13, or the salt, hydrate, or solvate thereof; the composition according to any one of claims 14 to 15; or the combination according to any one of claims 16 to 17; for use as a medicament.

19. The compound according to any one of claims 1 to 10, or the salt, hydrate, or solvate thereof; the conjugate according to any one of claims 11 to 13, or the salt, hydrate, or solvate thereof; the composition according to any one of claims 14 to 15; or the combination according to any one of claims 16 to 17; for use in the treatment of a disease in a subject,
preferably the subject is a human;
preferably the disease is cancer.

20. A method of treating a disease in a subject, wherein said method comprises the step of administering to said subject:
(a) the compound according to any one of claims 1 to 10, or the salt, hydrate, or solvate thereof;
(b) the conjugate according to any one of claims 11 to 13, or the salt, hydrate, or solvate thereof;
(c) the composition according to any one of claims 14 to 15; and/or
(d) the combination according to any one of claims 16 to 17;
preferably the subject is a human;
preferably the disease is cancer.

21. A non-therapeutic method for reacting:
(ia) the compound according to any one of claims 1 to 10, or the salt, hydrate, or solvate thereof;
(iia) the conjugate according to any one of claims 11 to 13, or the salt, hydrate, or solvate thereof; and/or
(iiia) the composition according to any one of claims 14 to 15;
with a diene or a salt, solvate, or hydrate thereof,
wherein said method comprises the step of contacting (ia), (iia), or (iiia) with said diene or salt, solvate, or hydrate thereof,
preferably said non-therapeutic method is an *in vitro* method; and
preferably said diene is a tetrazine.

22. A non-therapeutic use of:
(a) the compound according to any one of claims 1 to 10, or the salt, hydrate, or solvate thereof;
(b) the conjugate according to any one of claims 11 to 13, or the salt, hydrate, or solvate thereof;
(c) the composition according to any one of claims 14 to 15; and/or
(d) the combination according to any one of claims 16 to 17;
in a click reaction.
